# EUROPEAN PATENT APPLICATION

(11) **EP 4 653 533 A1**
(43) Date of publication of application: **26.11.2025**
(21) Application number: 24753393.8
(22) Date of filing: 07.02.2024
(51) Int. Cl.: C12N 15/13, A61K 39/395, A61K 47/68, A61P 25/00, C07K 14/52, C07K 14/765, C07K 16/18, C07K 19/00, C12N 1/15, C12N 1/19, C12N 1/21, C12N 5/10, C12N 15/62, C12N 15/63

(54) **HUMAN TRANSFERRIN RECEPTOR-AVID PEPTIDE**

(30) Priority: 07.02.2023 JP 2023016759
(71) Applicant: JCR Pharmaceuticals Co., Ltd., Ashiya-shi, Hyogo 659-0021 (JP)
(72) Inventor: ONOUCHI, Takashi, Kobe-shi, Hyogo 651-2241 (JP); TAKAHASHI, Kenichi, Kobe-shi, Hyogo 651-2241 (JP)
(74) Representative: Boult Wade Tennant LLP
(86) International application number: PCT/JP2024/004154
(87) International publication number: WO 2024/166950

(57) **Abstract**

Provided are a peptide having an affinity for a human transferrin receptor, which can be used for binding to any of compounds (a protein, a nucleic acid, a low-molecular-weight compound, and the like) that needs to exhibit its function in a central nervous system (CNS) and to be allowed to pass through a blood brain barrier, and a use thereof. The peptide is a peptide including, for example, an amino acid sequence selected from the group consisting of amino acid sequences set forth in SEQ ID NOs: 4 to 6, or a variant thereof.

## Description

### Technical Field

The present invention, in one embodiment, relates to a peptide having an affinity for a human transferrin receptor, which is used for binding to a compound (a protein, a nucleic acid, a low-molecular-weight compound, or the like) that needs to exhibit its function in a central nervous system (CNS) and to be allowed to pass through a blood brain barrier, and a use thereof.

### Background Art

Capillaries that supply blood to most of the brain tissues, except for several regions including the organs around the ventricles (pineal gland, pituitary gland, and posterior horn), are different from capillaries present in other tissues such as muscles in that endothelial cells forming the endothelium are closely adhered to each other by a strong intercellular adhesion. Therefore, passive transport of a substance from blood to the brain is hindered, and except for a fat-soluble substance or a substance having a small molecular weight (200 to 500 Dalton or less) and being electrically neutral in the vicinity of a physiological pH, it is difficult for the substance to migrate from a capillary to a brain parenchyma. Such a mechanism for restricting the substance exchange between the blood and the brain tissue fluid through the brain capillary endothelium is called a blood brain barrier (BBB). In addition, the blood brain barrier also restricts the substance exchange between the tissue fluid and the blood in the central nervous system including the brain and the spinal cord, in addition to the brain.

Due to the presence of the blood brain barrier, most of the cells of the central nervous system maintain their biochemical homeostasis without being affected by the concentration fluctuation of substances such as hormones and cytokines in the blood. However, the presence of the blood brain barrier raises a problem in the development of a drug. For example, for mucopolysaccharidosis type II (Hunter syndrome), which is a hereditary metabolic disorder caused by a deficiency of iduronate-2-sulfatase, enzyme replacement therapy of intravenously administering recombinant iduronate-2-sulfatase has been performed. However, for the significant abnormalities of central nervous system (CNS) observed in Hunter syndrome, the iduronate-2-sulfatase has low effectiveness because the iduronate-2-sulfatase cannot pass through the blood brain barrier. In addition, for a disease of the central nervous system such as Alzheimer's disease and brain tumor, it is necessary to allow a drug to reach the central nervous system, but a drug that cannot pass through the blood brain barrier cannot exhibit a drug efficacy.

Various methods for allowing such a polymer substance to be acted on the central nervous system such as a protein to pass through the blood brain barrier have been developed. As such a method, various methods of modifying a polymer substance to have an affinity for a membrane protein present on an endothelial cell of a brain capillary have been reported. Examples of the membrane protein present on the endothelial cell of the brain capillary include receptors for compounds such as insulin, transferrin, insulin-like growth factor (IGF-I, IGF-II), LDL, and leptin. Among these, with regard to the antibody against the transferrin receptor, the antibody which can pass through the blood brain barrier to migrate to the brain parenchyma and which binds a drug to be acted on the central nervous system and can allow the drug to migrate to the brain has been reported (Patent Literature 1 to 8).

Furthermore, an agent for treating Hunter syndrome (IZCARGO (registered trademark)) that can exert a drug efficacy in the central nervous system by passing through the blood brain barrier, in which iduronate-2-sulfatase is bound to an anti-transferrin receptor antibody, has been marketed in 2021 (Non-Patent Literature 1).

### Citation List

### Patent Literature

[Patent Literature 1] United States Patent Application, Publication No. 2007/0082380
[Patent Literature 2] United States Patent Application, Publication No. 2008/0152645
[Patent Literature 3] United States Patent Application, Publication No. 2009/0053219
[Patent Literature 4] United States Patent Application, Publication No. 2011/0110935
[Patent Literature 5] United States Patent Application, Publication No. 2018/0171012
[Patent Literature 6] United States Patent Application, Publication No. 2018/0179291
[Patent Literature 7] United States Patent Application, Publication No. 2020/0317798
[Patent Literature 8] United States Patent Application, Publication No. 2020/0384061

### Non Patent Literature

[Non-Patent Literature 1] Package Insert of IZCARGO (trade name) for I. V. infusion 10 mg, First Edition, created in March 2021

### Summary of Invention

### Technical problem

One object of the present invention, in the presence of a certain background, is to provide a peptide having an affinity for a human transferrin receptor, which can be used for binding to a compound (a protein, a nucleic acid, a low-molecular-weight compound, or the like) that needs to exhibit its function in a central nervous system (CNS) and to be allowed to pass through a blood brain barrier, and a use thereof.

### Solution to Problem

In the studies targeting the above-described object, the present inventors, as a result of a diligent study, have found that a peptide (anti-hTfR heavy chain antibody variable region peptide) obtained by a production method described in detail in the present specification, the peptide having an affinity for a human transferrin receptor (hTfR) and including a variable region of an anti-hTfR heavy chain antibody that specifically recognizes an extracellular region of hTfR, efficiently passes through the blood brain barrier, and have completed the present invention. That is, the present invention includes the following. In the invention shown in the following 1. to 104., the term "human transferrin receptor affinity peptide" can be replaced with a peptide including a variable region of a heavy chain antibody, and the term "peptide" can be replaced with a protein.
1. A peptide having an affinity for a human transferrin receptor (a human transferrin receptor affinity peptide), comprising a variable region of a heavy chain antibody having three complementary determining regions of CDR1, CDR2, and CDR3,
   wherein the peptide is selected from the group consisting of the following (1) and (2),
   (1) a peptide in which the amino acid sequence of CDR1 is the amino acid sequence set forth in SEQ ID NO: 7 or SEQ ID NO: 8, the amino acid sequence of CDR2 is the amino acid sequence set forth in SEQ ID NO: 9 or SEQ ID NO: 10, and the amino acid sequence of CDR3 is the amino acid sequence set forth in SEQ ID NO: 11 or SEQ ID NO: 12, and
   (2) a peptide in which the amino acid sequence of CDR1 is the amino acid sequence set forth in SEQ ID NO: 13 or SEQ ID NO: 14, the amino acid sequence of CDR2 is the amino acid sequence set forth in SEQ ID NO: 15 or SEQ ID NO: 16, and the amino acid sequence of CDR3 is the amino acid sequence set forth in SEQ ID NO: 17 or SEQ ID NO: 18.
2. A human transferrin receptor affinity peptide, which is a peptide selected from the group consisting of the following (1) to (3),
   (1) a peptide having 80% or more amino acid sequence identity to the amino acid sequence set forth in SEQ ID NO: 4, and having three complementary determining regions of CDR1, CDR2, and CDR3, which have the amino acid sequences shown in (1) of above-described 1.,
   (2) a peptide having 80% or more amino acid sequence identity to the amino acid sequence set forth in SEQ ID NO: 5, and having three complementary determining regions of CDR1, CDR2, and CDR3, which have the amino acid sequences shown in (1) of above-described 1., and
   (3) a peptide having 80% or more amino acid sequence identity to the amino acid sequence set forth in SEQ ID NO: 6, and having three complementary determining regions of CDR1, CDR2, and CDR3, which have the amino acid sequences shown in (2) of above-described 1.
3. The human transferrin receptor affinity peptide according to above-described 2., wherein the amino acid sequence identity is 85% or more.
4. The human transferrin receptor affinity peptide according to above-described 2., wherein the amino acid sequence identity is 90% or more.
5. The human transferrin receptor affinity peptide according to above-described 2., wherein the amino acid sequence identity is 95% or more.
6. The human transferrin receptor affinity peptide according to above-described 1., wherein the peptide includes the amino acid sequence selected from the group consisting of (1) to (3),
   (1) an amino acid sequence in which 1 to 10 amino acids in the amino acid sequence set forth in SEQ ID NO: 4 are substituted, deleted, or added, and in which the amino acid sequence of CDR1 is the amino acid sequence set forth in SEQ ID NO: 7 or SEQ ID NO: 8, the amino acid sequence of CDR2 is the amino acid sequence set forth in SEQ ID NO: 9 or SEQ ID NO: 10, and the amino acid sequence of CDR3 is the amino acid sequence set forth in SEQ ID NO: 11 or SEQ ID NO: 12,
   (2) an amino acid sequence in which 1 to 10 amino acids in the amino acid sequence set forth in SEQ ID NO: 5 are substituted, deleted, or added, and in which the amino acid sequence of CDR1 is the amino acid sequence set forth in SEQ ID NO: 7 or SEQ ID NO: 8, the amino acid sequence of CDR2 is the amino acid sequence set forth in SEQ ID NO: 9 or SEQ ID NO: 10, and the amino acid sequence of CDR3 is the amino acid sequence set forth in SEQ ID NO: 11 or SEQ ID NO: 12, and
   (3) an amino acid sequence in which 1 to 10 amino acids in the amino acid sequence set forth in SEQ ID NO: 6 are substituted, deleted, or added, and in which the amino acid sequence of CDR1 is the amino acid sequence set forth in SEQ ID NO: 13 or SEQ ID NO: 14, the amino acid sequence of CDR2 is the amino acid sequence set forth in SEQ ID NO: 15 or SEQ ID NO: 16, and the amino acid sequence of CDR3 is the amino acid sequence set forth in SEQ ID NO: 17 or SEQ ID NO: 18.
7. The human transferrin receptor affinity peptide according to above-described 6., whereinthe number of substituted, deleted, or added amino acids is 1 to 5.
8. The human transferrin receptor affinity peptide according to above-described 6., whereinthe number of substituted, deleted, or added amino acids is 1 to 3.
9. The human transferrin receptor affinity peptide according to any one of above-described 1. to 8., wherein the peptide has an affinity for both an extracellular region of a human transferrin receptor and an extracellular region of a monkey transferrin receptor.
10. The human transferrin receptor affinity peptide according to above-described 9., wherein both a dissociation constant with the extracellular region of the human transferrin receptor and a dissociation constant with the extracellular region of the monkey transferrin receptor are 5 × 10⁻¹¹ M to 5 × 10⁻⁷ M.
11. The human transferrin receptor affinity peptide according to above-described 9., wherein both of dissociation constants with the extracellular region of the human transferrin receptor are all 5 × 10⁻¹¹ M to 1 × 10⁻⁸ M.
12. The human transferrin receptor affinity peptide according to above-described 9., wherein in a case where a value of a dissociation constant with the extracellular region of the human transferrin receptor is designated as 1, a value of a dissociation constant with the monkey transferrin receptor is 1 to 10.
13. The human transferrin receptor affinity peptide according to above-described 9., wherein in a case where a value of a dissociation constant with the extracellular region of the human transferrin receptor is designated as 1, a value of a dissociation constant with the monkey transferrin receptor is 3 to 10.
14. A human transferrin receptor affinity peptide in which a plurality of the human transferrin receptor affinity peptides according to any one of above-described 1. to 13. are bound, wherein the peptide is selected from the group consisting of the following (1) to (3),
   (1) a peptide in which 2 to 10 of the human transferrin receptor affinity peptides according to any one of above-described 1. to 12. are, directly or via a linker, bound,
   (2) a peptide in which 2 or 3 of the human transferrin receptor affinity peptides according to any one of above-described 1. to 12. are, directly or via a linker, bound, and
   (3) a peptide in which 2 of the human transferrin receptor affinity peptides according to any one of above-described 1. to 12. are, directly or via a linker, bound.
15. The human transferrin receptor affinity peptide according to above-described 14., wherein each of linkers which link a plurality of the human transferrin receptor affinity peptides to each other is a peptide linker consisting of 1 to 60 amino acids.
16. The human transferrin receptor affinity peptide according to above-described 14., wherein the amino acid sequence of the peptide linker is selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, SEQ ID NO: 21, SEQ ID NO: 22, SEQ ID NO: 23, SEQ ID NO: 24, and SEQ ID NO: 25.
17. The human transferrin receptor affinity peptide according to above-described 14., wherein the amino acid sequence of the peptide linker consists of the amino acid sequence in which 2 to 10 amino acid sequences each selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, and SEQ ID NO: 21 are arranged in series.
18. The human transferrin receptor affinity peptide according to above-described 1., wherein the peptide includes the amino acid sequence selected from the group consisting of the amino acid sequences set forth in SEQ ID NO: 4, SEQ ID NO: 5, and SEQ ID NO: 6.
19. A human transferrin receptor affinity peptide which includes the amino acid sequence in which 1 to 10 amino acids in the amino acid sequence selected from the group consisting of the amino acid sequences set forth in SEQ ID NO: 4, SEQ ID NO: 5, and SEQ ID NO: 6 are substituted, deleted, or added.
20. The human transferrin receptor affinity peptide according to above-described 37., wherein the number of substituted, deleted, or added amino acids is 1 to 5.
21. The human transferrin receptor affinity peptide according to above-described 37., wherein the number of substituted, deleted, or added amino acids is 1 to 3.
22. A human transferrin receptor affinity peptide-drug complex in which a drug is bound to the human transferrin receptor affinity peptide according to any one of above-described 1. to 21.
23. The complex according to above-described 22., wherein the drug is any of a different protein (A), a nucleic acid, or a low-molecular-weight compound.
24. A fusion protein of the human transferrin receptor affinity peptide according to any one of above-described 1. to 21. and a different protein (A).
25. The fusion protein according to above-described 24., in which the human transferrin receptor affinity peptide is bound to the C-terminus of the different protein (A) directly or via a linker.
26. The fusion protein according to above-described 24., in which the human transferrin receptor affinity peptide is bound to the N-terminus of the different protein (A) directly or via a linker.
27. The fusion protein according to above-described 25. or 26., wherein the linker that links the human transferrin receptor affinity peptide to the different protein (A) is a peptide linker consisting of 1 to 60 amino acids.
28. The fusion protein according to above-described 27., wherein the amino acid sequence of the peptide linker that links the human transferrin receptor affinity peptide to the different protein (A) is selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, SEQ ID NO: 21, SEQ ID NO: 22, SEQ ID NO: 23, SEQ ID NO: 24, and SEQ ID NO: 25.
29. The fusion protein according to above-described 27., wherein the amino acid sequence of the peptide linker that links the human transferrin receptor affinity peptide to the different protein (A) consists of the amino acid sequence in which 2 to 10 amino acid sequences each selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, and SEQ ID NO: 21 are arranged in series.
30. The fusion protein according to any one of above-described 24. to 29., wherein the different protein (A) is a protein derived from a human.
31. The fusion protein according to any one of above-described 24. to 30., wherein the different protein (A) is a cytokine, a growth factor, or an antibody drug.
32. The fusion protein according to any one of above-described 24. to 30., wherein the different protein (A) is selected from the group consisting of a brain-derived nerve growth factor (BDNF), a nerve growth factor (NGF), a lysosomal enzyme, a ciliary neurotrophic factor (CNTF), a glial cell line neurotrophic factor (GDNF), a neurotrophin 3, a neurotrophin 4/5, a neurotrophin 6, a neuregulin 1, an erythropoietin, a darbepoietin, an activin, a basic fibroblast growth factor (bFGF), a fibroblast growth factor 2 (FGF2), an epidermal growth factor (EGF), a vascular endothelial growth factor (VEGF), an interferon α, an interferon β, an interferon γ, an interleukin 6, a granulocyte-macrophage colony-stimulating factor (GM-CSF), a granulocyte colony-stimulating factor (G-CSF), a macrophage colony-stimulating factor (M-CSF), a tumor necrosis factor α receptor (TNF-α receptor), a PD-1 ligand, a PD-L1, a PD-L2, an enzyme having an activity of degrading β-amyloid, an anti-β-amyloid antibody, an anti-BACE antibody, an anti-EGFR antibody, an anti-PD-1 antibody, an anti-PD-L1 antibody, an anti-PD-L2 antibody, an anti-HER2 antibody, an anti-TNF-α antibody, and an anti-CTLA-4 antibody.
33. The fusion protein according to any one of above-described 24. to 30., wherein the different protein (A) is a lysosomal enzyme, and the lysosomal enzyme is selected from the group consisting of α-L-iduronidase, iduronate-2-sulfatase, acidic α-glucosidase, glucocerebrosidase, β-galactosidase, GM2-activator protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acidic sphingomyelinase, α-galactosidase A, β-glucuronidase, heparan N-sulfatase, α-N-acetylglucosaminidase, acetyl CoA α-glucosaminide N-acetyltransferase, N-acetylglucosamine-6-sulfatase, acidic ceramidase, amylo-1,6-glucosidase, sialidase, palmitoyl-protein thioesterase-1, tripeptidyl peptidase-1, hyaluronidase-1, CLN1, and CLN2.
34. The fusion protein according to any one of above-described 24. to 33., wherein serum albumin is bound to the fusion protein.
35. The fusion protein according to any one of above-described 24. to 33., wherein a human IgG Fc region or a part thereof is bound to the fusion protein.
36. A nucleic acid encoding the human transferrin receptor affinity peptide according to any one of above-described 1. to 21.
37. A nucleic acid encoding the fusion protein according to any one of above-described 24. to 35.
38. An expression vector comprising the nucleic acid according to above-described 36. or 37.
39. A cell transformed with the expression vector according to above-described 38.
40. The cell according to above-described 39., wherein the cell is derived from a mammal.
41. The human transferrin receptor affinity peptide according to above-described 1., which is selected from the group consisting of the following (1) to (3),
   (1) a peptide having 80% or more identity to the amino acid sequence set forth in SEQ ID NO: 4, in which the amino acid sequence of CDR1 is the amino acid sequence set forth in SEQ ID NO: 7 or SEQ ID NO: 8, the amino acid sequence of CDR2 is the amino acid sequence set forth in SEQ ID NO: 9 or SEQ ID NO: 10, and the amino acid sequence of CDR3 is the amino acid sequence set forth in SEQ ID NO: 11 or SEQ ID NO: 12,
   (2) a peptide having 80% or more identity to the amino acid sequence set forth in SEQ ID NO: 5, in which the amino acid sequence of CDR1 is the amino acid sequence set forth in SEQ ID NO: 7 or SEQ ID NO: 8, the amino acid sequence of CDR2 is the amino acid sequence set forth in SEQ ID NO: 9 or SEQ ID NO: 10, and the amino acid sequence of CDR3 is the amino acid sequence set forth in SEQ ID NO: 11 or SEQ ID NO: 12, and
   (3) a peptide having 80% or more identity to the amino acid sequence set forth in SEQ ID NO: 6, in which the amino acid sequence of CDR1 is the amino acid sequence set forth in SEQ ID NO: 13 or SEQ ID NO: 14, the amino acid sequence of CDR2 is the amino acid sequence set forth in SEQ ID NO: 15 or SEQ ID NO: 16, and the amino acid sequence of CDR3 is the amino acid sequence set forth in SEQ ID NO: 17 or SEQ ID NO: 18.
42. The human transferrin receptor affinity peptide according to above-described 41., wherein the amino acid sequence identity is 85% or more.
43. The human transferrin receptor affinity peptide according to above-described 41., wherein the amino acid sequence identity is 90% or more.
44. The human transferrin receptor affinity peptide according to above-described 41., wherein the amino acid sequence identity is 95% or more.
45. A human transferrin receptor affinity peptide-drug complex in which a drug is bound to the human transferrin receptor affinity peptide according to any one of above-described 37. to 44.
46. The complex according to above-described 45., wherein the drug is any of a different protein (A), a nucleic acid, or a low-molecular-weight compound.
47. A fusion protein of a human transferrin receptor affinity peptide, which has an affinity for a molecule present on a surface of a vascular endothelial cell as an antigen, and a different protein (A), wherein the human transferrin receptor affinity peptide is bound to the C-terminus of the different protein (A) directly or via a linker, or wherein the human transferrin receptor affinity peptide is bound to the N-terminus of the different protein (A) directly or via a linker.
48. The fusion protein according to above-described 47., wherein the linker is a peptide linker consisting of 1 to 60 amino acids.
49. The fusion protein according to above-described 48., wherein the amino acid sequence of the peptide linker is selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, SEQ ID NO: 21, SEQ ID NO: 22, SEQ ID NO: 23, SEQ ID NO: 24, and SEQ ID NO: 25.
50. The fusion protein according to above-described 48., wherein the amino acid sequence of the peptide linker consists of the amino acid sequence in which 2 to 10 amino acid sequences each selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, and SEQ ID NO: 21 are arranged in series.
51. The complex or fusion protein according to any one of above-described 46. to 50., wherein the different protein (A) is a protein derived from a human.
52. The complex or fusion protein according to any one of above-described 46. to 51., wherein the different protein (A) is a cytokine, a growth factor, or an antibody drug.
53. The complex or fusion protein according to any one of above-described 46. to 51., wherein the different protein (A) is selected from the group consisting of a brain-derived nerve growth factor (BDNF), a nerve growth factor (NGF), a lysosomal enzyme, a ciliary neurotrophic factor (CNTF), a glial cell line neurotrophic factor (GDNF), a neurotrophin 3, a neurotrophin 4/5, a neurotrophin 6, a neuregulin 1, an erythropoietin, a darbepoietin, an activin, a basic fibroblast growth factor (bFGF), a fibroblast growth factor 2 (FGF2), an epidermal growth factor (EGF), a vascular endothelial growth factor (VEGF), an interferon α, an interferon β, an interferon γ, an interleukin 6, a granulocyte-macrophage colony-stimulating factor (GM-CSF), a granulocyte colony-stimulating factor (G-CSF), a macrophage colony-stimulating factor (M-CSF), a tumor necrosis factor α receptor (TNF-α receptor), a PD-1 ligand, a PD-L1, a PD-L2, an enzyme having an activity of degrading β-amyloid, an anti-β-amyloid antibody, an anti-BACE antibody, an anti-EGFR antibody, an anti-PD-1 antibody, an anti-PD-L1 antibody, an anti-PD-L2 antibody, an anti-HER2 antibody, an anti-TNF-α antibody, and an anti-CTLA-4 antibody.
54. The complex or fusion protein according to any one of above-described 46. to 51., wherein the different protein (A) is a lysosomal enzyme, and the lysosomal enzyme is selected from the group consisting of α-L-iduronidase, iduronate-2-sulfatase, acidic α-glucosidase, glucocerebrosidase, β-galactosidase, GM2-activator protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acidic sphingomyelinase, α-galactosidase A, β-glucuronidase, heparan N-sulfatase, α-N-acetylglucosaminidase, acetyl CoA α-glucosaminide N-acetyltransferase, N-acetylglucosamine-6-sulfatase, acidic ceramidase, amylo-1,6-glucosidase, sialidase, palmitoyl-protein thioesterase-1, tripeptidyl peptidase-1, hyaluronidase-1, CLN1, and CLN2.
55. The fusion protein according to any one of above-described 46. to 54., wherein serum albumin is further bound to the fusion protein.
56. The fusion protein according to any one of above-described 46. to 54., wherein a human IgG Fc region or a part thereof is further bound to the fusion protein.
57. The complex or fusion protein according to any one of above-described 45. to 56., wherein the vascular endothelial cell is a brain vascular endothelial cell.
58. The complex or fusion protein according to any one of above-described 45. to 57., wherein the molecule present on a surface of the vascular endothelial cell is selected from the group consisting of a transferrin receptor (TfR), an insulin receptor, a leptin receptor, a lipoprotein receptor, an IGF receptor, OATP-F, an organic anion transporter, MCT-8, and a monocarboxylate transporter, and is particularly a molecule derived from a human.
59. The complex or fusion protein according to any one of above-described 45. to 57., wherein the molecule present on a surface of the vascular endothelial cell is a transferrin receptor (TfR), particularly a transferrin receptor derived from a human.
60. The complex according to above-described 45. or 46., wherein the drug is bound to the human transferrin receptor affinity peptide via a linker selected from the group consisting of a polyethylene glycol, a polypropylene glycol, a copolymer of ethylene glycol and propylene glycol, a polyoxyethylated polyol, a polyvinyl alcohol, a polysaccharide, a dextran, a polyvinyl ether, a biodegradable polymer, a lipid polymer, a chitin, a hyaluronic acid, biotin-streptavidin, and a derivative thereof.
61. The complex or fusion protein according to any one of above-described 45. to 60., wherein the human transferrin receptor affinity peptide is all or a part of a heavy chain antibody derived from a camelidae animal or an immunoglobulin new antigen receptor derived from cartilaginous fishes.
62. The complex or fusion protein according to above-described 61., wherein the heavy chain antibody is a single heavy chain antibody.
63. The complex or fusion protein according to above-described 61. or 62., wherein the heavy chain antibody is selected from the group consisting of the following (1) to (4),
   (1) a heavy chain antibody consisting of all or a part of a variable region of a heavy chain,
   (2) a heavy chain antibody consisting of all or a part of the variable region and a part of a hinge region of the heavy chain,
   (3) a heavy chain antibody consisting of all of the variable region and the hinge region of the heavy chain, and
   (4) a heavy chain antibody consisting of a part of a constant region of the heavy chain in addition to the variable region and the hinge region of the heavy chain.
64. A nucleic acid encoding the fusion protein according to any one of above-described 47. to 59., or 61. to 63.
65. An expression vector including the nucleic acid according to above-described 64.
66. A cell transformed with the expression vector according to above-described 65.
67. The cell according to above-described 66., wherein the cell is derived from a mammal.
68. A pharmaceutical composition comprising the complex or fusion protein of any one of above-described 45. to 63. as an active ingredient, particulary wherein the active ingredient is the drug that exhibits a drug efficacy in the central nervous system by passing through a blood brain barrier.
69. A heavy chain antibody variable region peptide having affinity for a human transferrin receptor, the peptide comprising three complementary determining regions of CDR1, CDR2, and CDR3, wherein the peptide is selected from the group consisting of the following (1) and (2),
   (1) a peptide in which the amino acid sequence of CDR1 is the amino acid sequence set forth in SEQ ID NO: 7 or SEQ ID NO: 8, the amino acid sequence of CDR2 is the amino acid sequence set forth in SEQ ID NO: 9 or SEQ ID NO: 10, and the amino acid sequence of CDR3 is the amino acid sequence set forth in SEQ ID NO: 11 or SEQ ID NO: 12, where a combination of the amino acid sequences of CDR1, CDR2, and CDR3 may be any combination, but is preferably a combination in which the amino acid sequence of CDR1 is the amino acid sequence set forth in SEQ ID NO: 7, the amino acid sequence of CDR2 is the amino acid sequence set forth in SEQ ID NO: 9, and the amino acid sequence of CDR3 is the amino acid sequence set forth in SEQ ID NO: 11, or a combination in which the amino acid sequence of CDR1 is the amino acid sequence set forth in SEQ ID NO: 8, the amino acid sequence of CDR2 is the amino acid sequence set forth in SEQ ID NO: 10, and the amino acid sequence of CDR3 is the amino acid sequence set forth in SEQ ID NO: 12, and
   (2) a peptide in which the amino acid sequence of CDR1 is the amino acid sequence set forth in SEQ ID NO: 13 or SEQ ID NO: 14, the amino acid sequence of CDR2 is the amino acid sequence set forth in SEQ ID NO: 15 or SEQ ID NO: 16, and the amino acid sequence of CDR3 is the amino acid sequence set forth in SEQ ID NO: 17 or SEQ ID NO: 18, where a combination of the amino acid sequences of CDR1, CDR2, and CDR3 may be any combination, but is preferably a combination in which the amino acid sequence of CDR1 is the amino acid sequence set forth in SEQ ID NO: 13, the amino acid sequence of CDR2 is the amino acid sequence set forth in SEQ ID NO: 15, and the amino acid sequence of CDR3 is the amino acid sequence set forth in SEQ ID NO: 17, or a combination in which the amino acid sequence of CDR1 is the amino acid sequence set forth in SEQ ID NO: 14, the amino acid sequence of CDR2 is the amino acid sequence set forth in SEQ ID NO: 16, and the amino acid sequence of CDR3 is the amino acid sequence set forth in SEQ ID NO: 18.
70. A heavy chain antibody variable region peptide having affinity for a human transferrin receptor, the peptide comprising three complementary determining regions of CDR1, CDR2, and CDR3, wherein the peptide is selected from the group consisting of the following (1) to (3),
   (1) a peptide having 80% or more identity to the amino acid sequence set forth in SEQ ID NO: 4, in which the amino acid sequences of CDR1, CDR1, and CDR3 are shown in (1) of above-described 69.,
   (2) a peptide having 80% or more identity to the amino acid sequence set forth in SEQ ID NO: 5, in which the amino acid sequences of CDR1, CDR1, and CDR3 are shown in (1) of above-described 69., and
   (3) a peptide having 80% or more identity to the amino acid sequence set forth in SEQ ID NO: 6, in which the amino acid sequences of CDR1, CDR1, and CDR3 are shown in (2) of above-described 69.
71. The heavy chain antibody variable region peptide selected from the group consisting of (1) to (3) of above-described 70., wherein the identity is 85% or more.
72. The heavy chain antibody variable region peptide selected from the group consisting of (1) to (3) of above-described 70., wherein the identity is 90% or more.
73. The heavy chain antibody variable region peptide selected from the group consisting of (1) to (3) of above-described 70., wherein the identity is 95% or more.
74. A heavy chain antibody variable region peptide having affinity for a human transferrin receptor, the peptide comprising three complementary determining regions of CDR1, CDR2, and CDR3, wherein the peptide is selected from the group consisting of the following (1) to (3),
   (1) a peptide including the amino acid sequence in which 1 to 10 amino acids in the amino acid sequence set forth in SEQ ID NO: 4 are substituted, deleted, or added, and in which the amino acid sequences of CDR1, CDR1, and CDR3 are shown in (1) of above-described 69.,
   (2) a peptide including the amino acid sequence in which 1 to 10 amino acids in the amino acid sequence set forth in SEQ ID NO: 5 are substituted, deleted, or added, and in which the amino acid sequences of CDR1, CDR1, and CDR3 are shown in (1) of above-described 69., and
   (3) a peptide including the amino acid sequence in which 1 to 10 amino acids in the amino acid sequence set forth in SEQ ID NO: 6 are substituted, deleted, or added, and in which the amino acid sequences of CDR1, CDR1, and CDR3 are shown in (2) of above-described 69.
75. The heavy chain antibody variable region peptide selected from the group consisting of (1) to (3) of above-described 74., wherein a number of substituted, deleted, or added amino acids is 1 to 5.
76. The heavy chain antibody variable region peptide selected from the group consisting of (1) to (3) of above-described 74., wherein a number of substituted, deleted, or added amino acids is 1 to 3.
77. The heavy chain antibody variable region peptide according to any one of above-described 69. to 76., wherein the peptide has an affinity for both an extracellular region of a human transferrin receptor and an extracellular region of a monkey transferrin receptor.
78. The heavy chain antibody variable region peptide according to above-described 77., wherein both a dissociation constant with the extracellular region of the human transferrin receptor and a dissociation constant with the extracellular region of the monkey transferrin receptor are 5 × 10⁻¹¹ M to 5 × 10⁻⁷ M.
79. The heavy chain antibody variable region peptide according to above-described 77., wherein both a dissociation constant with the extracellular region of the human transferrin receptor and a dissociation constant with the extracellular region of the monkey transferrin receptor are 5 × 10⁻¹¹ M to 1 × 10⁻⁸ M.
80. The heavy chain antibody variable region peptide according to above-described 77., wherein in a case where a value of a dissociation constant with the extracellular region of the human transferrin receptor is designated as 1, a value of a dissociation constant with a monkey transferrin receptor is 1 to 10.
81. The heavy chain antibody variable region peptide according to above-described 77., wherein in a case where a value of a dissociation constant with the extracellular region of the human transferrin receptor is designated as 1, a value of a dissociation constant with a monkey transferrin receptor is 3 to 10.
82. A heavy chain antibody variable region peptide in which a plurality of the heavy chain antibody variable region peptides according to any one of above-described 69. to 81. are bound, wherein the peptide is selected from the group consisting of the following (1) to (3),
   (1) a peptide in which 2 to 10 of the heavy chain antibody variable region peptides according to above-described 41. to 53. are, directly or via a linker, bound,
   (2) a peptide in which 2 or 3 of the heavy chain antibody variable region peptides according to above-described 41. to 53. are, directly or via a linker, bound, and
   (3) a peptide in which 2 of the heavy chain antibody variable region peptides according to above-described 41. to 53. are, directly or via a linker, bound.
83. The heavy chain antibody variable region peptide according to above-described 82., wherein each of linkers which link a plurality of the heavy chain antibody variable region peptides to each other is a peptide linker consisting of 1 to 60 amino acids.
84. The heavy chain antibody variable region peptide according to above-described 83., wherein the amino acid sequence of the peptide linker is selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, SEQ ID NO: 21, SEQ ID NO: 22, SEQ ID NO: 23, SEQ ID NO: 24, and SEQ ID NO: 25.
85. The heavy chain antibody variable region peptide, wherein the amino acid sequence of the peptide linker consists of the amino acid sequence in which 2 to 10 amino acid sequences each selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, and SEQ ID NO: 21 are arranged in series.
86. A heavy chain antibody variable region peptide-drug complex in which a heavy chain antibody variable region peptide and a drug are bound to each other, wherein the heavy chain antibody variable region peptide is the peptide according to any one of above-described 69. to 85.
87. The complex according to above-described 86., wherein the drug is any of a different protein (A), a nucleic acid, or a low-molecular-weight compound.
88. A fusion protein of a heavy chain antibody variable region peptide and a different protein (A), wherein the heavy chain antibody variable region peptide is the peptide according to any one of above-described 69. to 85.
89. The fusion protein according to above-described 88., wherein the heavy chain antibody variable region peptide is bound to the C-terminus of the different protein (A) directly or via a linker.
90. The fusion protein according to above-described 88., wherein the heavy chain antibody variable region peptide is bound to the N-terminus of the different protein (A) directly or via a linker.
91. The fusion protein according to above-described 89. or 90., wherein the linker is a peptide linker consisting of 1 to 60 amino acids.
92. The fusion protein according to above-described 91., wherein the amino acid sequence of the peptide linker is selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, SEQ ID NO: 21, SEQ ID NO: 22, SEQ ID NO: 23, SEQ ID NO: 24, and SEQ ID NO: 25.
93. The fusion protein according to above-described 91., wherein the amino acid sequence of the peptide linker consists of the amino acid sequence in which 2 to 10 amino acid sequences each selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, and SEQ ID NO: 21 are arranged in series.
94. The complex or fusion protein according to any one of above-described 87. to 93., wherein the different protein (A) is a protein derived from a human.
95. The complex or fusion protein according to any one of above-described 87. to 94., wherein the different protein (A) is a cytokine, a growth factor, or an antibody drug.
96. The complex or fusion protein according to any one of above-described 87. to 94., wherein the different protein (A) is selected from the proteins shown in above-described 35.
97. The complex or fusion protein according to any one of above-described 87. to 94., wherein the different protein (A) is a lysosomal enzyme, and the lysosomal enzyme is selected from the enzymes shown in above-described 36.
98. The complex or fusion protein according to any one of above-described 87. to 94., wherein serum albumin is bound to the complex or fusion protein.
99. The complex or fusion protein according to any one of above-described 87. to 94., wherein a human IgG Fc region or a part thereof is bound to the fusion protein.
100. A nucleic acid encoding the heavy chain antibody variable region peptide according to any one of above-described 69. to 85.
101. A nucleic acid encoding the fusion proteins according to any one of above-described 88. to 99.
102. An expression vector including the nucleic acid according to above-described 100. or 101.
103. A cell transformed with the expression vector according to above-described 102.
104. The cell according to above-described 103., wherein the cell is derived from a mammal.

### Advantageous Effects of Invention

The peptide (hTfR affinity peptide) having an affinity for the human transferrin receptor according to one embodiment of the present invention can be bound to a substance that can hardly or cannot pass through the blood brain barrier, and can allow the substance to pass through the blood brain barrier and reach the central nervous system. Therefore, the function of the substance can be exhibited in the central nervous system.

### Brief Description of Drawings

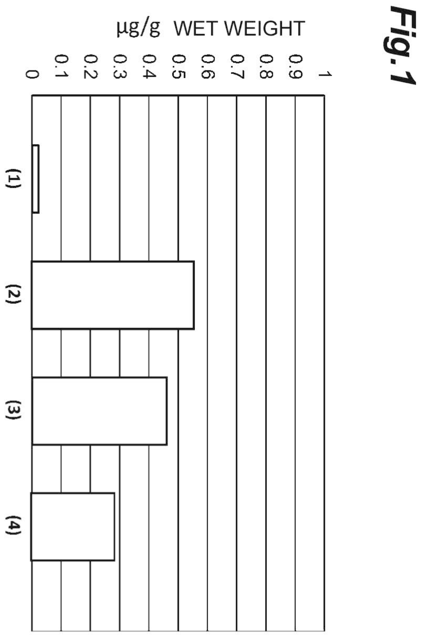

Fig. 1 is a graph showing quantitative values of the hTfR affinity peptide-trastuzumab and trastuzumab contained in the cerebellum of a wild-type mouse 6 hours after the administration of each of the hTfR affinity peptide-trastuzumab and the trastuzumab. (1) represents the quantitative value of trastuzumab, (2) represents the quantitative value of hTfR affinity peptide-trastuzumab 1, (3) represents the quantitative value of hTfR affinity peptide-trastuzumab 3, and (4) represents the quantitative value of hTfR affinity peptide-trastuzumab (reference product). The vertical axis indicates the amount (µg) of these substances contained per wet weight (g).

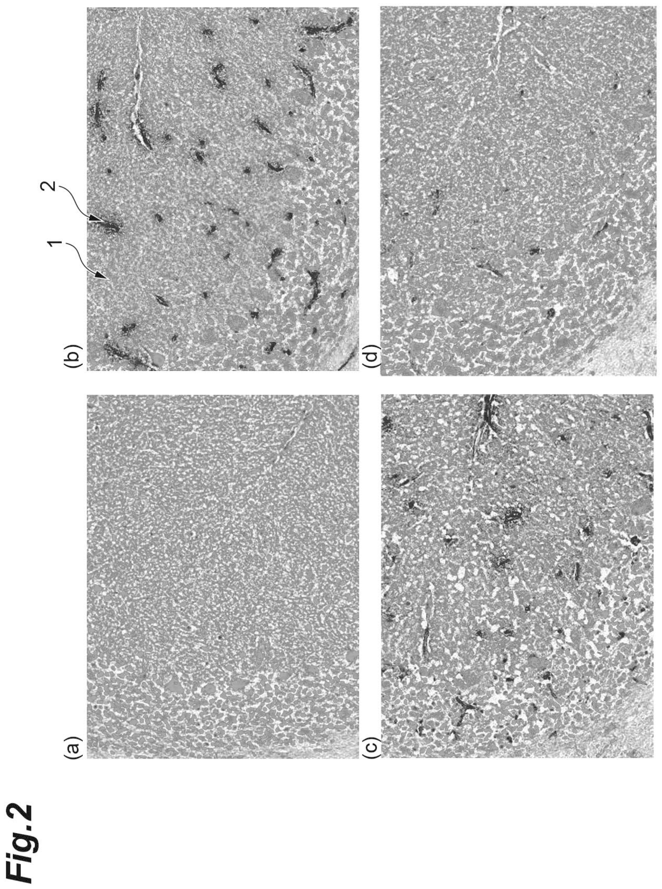

Fig. 2 is a photograph in substitution for a drawing showing the result of immunohistochemical staining for an anti-hTfR antibody of a cerebellum of a wild-type mouse 6 hours after the administration of the hTfR affinity peptide-trastuzumab. (a) shows the result of administration of trastuzumab, (b) shows the result of administration of hTfR affinity peptide-trastuzumab 1, (c) shows the result of administration of hTfR affinity peptide-trastuzumab 3, and (d) shows the result of administration of hTfR affinity peptide-trastuzumab (reference product). Each photograph has been taken at a magnification of 40X.

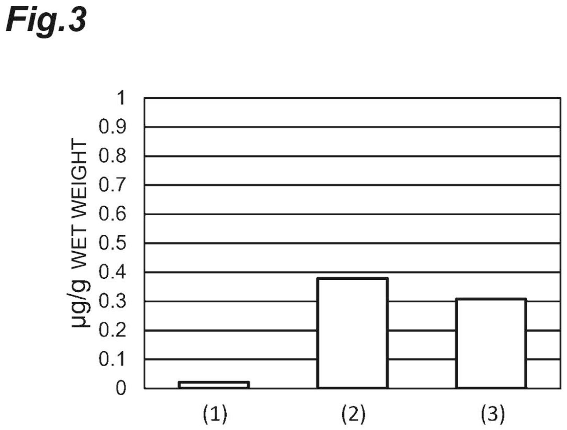

Fig. 3 is a graph showing quantitative values of the hTfR affinity peptide-trastuzumab and trastuzumab contained in the cerebellum of a hTfR knock-in mouse 6 hours after the administration of each of the hTfR affinity peptide-trastuzumab and the trastuzumab. (1) represents the quantitative value of trastuzumab, (2) represents the quantitative value of hTfR affinity peptide-trastuzumab 2, and (3) represents the quantitative value of hTfR affinity peptide-trastuzumab 3. The vertical axis indicates the amount (µg) of these substances contained per wet weight (g).

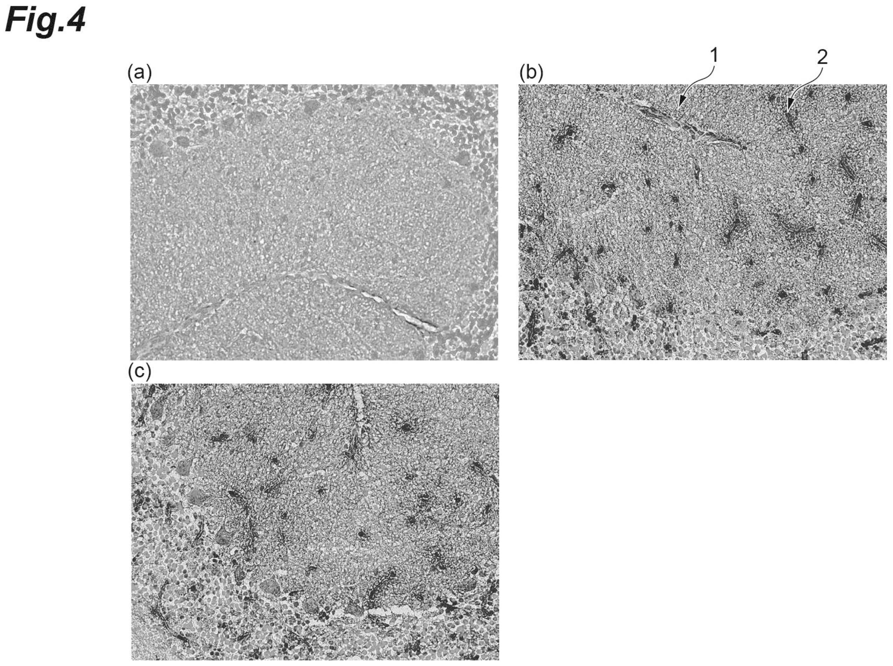

Fig. 4 is a photograph in substitution for a drawing showing the result of immunohistochemical staining for an anti-hTfR antibody of a cerebellum of the hTfR knock-in mouse 6 hours after the administration of the hTfR affinity peptide-trastuzumab. (a) shows the result of administration of trastuzumab, (b) shows the result of administration of hTfR affinity peptide-trastuzumab 2, and (c) shows the result of administration of hTfR affinity peptide-trastuzumab 3. Each photograph has been taken at a magnification of 40X.

### Description of Embodiments

A monomer of an antibody of many mammals including a human and a mouse is formed as a heterotetramer in which two H chains and two L chains are covalently bound to each other by a disulfide bond. Each chain has a region in which the amino acid sequences of the different antibodies differ at the N-terminus, and this region is referred to as a variable region. In the variable region of the H chain, there are three complementary determining regions that significantly contribute to the specific binding to an antigen. The three complementary determining regions are referred to as CDR1, CDR2, and CDR3 from the N-terminus. Similarly, in the L chain, there are three complementary determining regions that significantly contribute to the specific binding with the antigen, and these three complementary determining regions are referred to as CDR1, CDR2, and CDR3 from the N-terminus.

One H chain and one L chain form a pair and specifically bind to an antigen. That is, the specificity with the antigen is substantially determined by the amino acid sequences of the 6 CDRs of the 3 CDRs of the H chain and the 3 CDRs of the L chain. Since the monomer of the antibody of many mammals has two pairs of an H chain and an L chain, the molecule of one antibody has two regions that can bind to the antigen.

In the variable region, a region other than the CDR is referred to as a framework region (FR). The H chain and the L chain each have four FRs, which are referred to as FR1, FR2, FR3, and FR4 from the N-terminus. The CDR is present between the FR in the variable region.

FR1 is a region from the N-terminus of a variable region of the heavy chain antibody to an amino acid adjacent to the N-terminus of CDR1. FR2 is a region between CDR1 and CDR2. FR3 is a region between CDR2 and CDR3. FR4 is a region from an amino acid adjacent to the C-terminus of CDR3 to the C-terminus of the variable region of the heavy chain antibody.

With regard to the monomer of the above-described antibodies of many mammals (normal antibodies), there is an antibody (heavy chain antibody) that does not have an L chain and includes only a heavy chain. For example, a heavy chain antibody (hcIg) produced by a camelidae animal and an immunoglobulin new antigen receptor (IgNAR) produced by cartilaginous fishes including a shark correspond to this. A camelidae heavy chain antibody (hcIg) is a homodimer in which two heavy chains are covalently bound to each other by a disulfide bond. Each heavy chain has a variable region at the N-terminus, and the variable region has three complementary determining regions (CDRs) that significantly contribute to the specific binding with an antigen. The three complementary determining regions are referred to as CDR1, CDR2, and CDR3 from the N-terminus. Since each heavy chain constituting the hcIg can specifically bind to an antigen even though it is a monomer, one hcIg has two regions in which an antigen can be bound in a molecule. In the variable region, a region other than the CDR is referred to as a framework region (FR), and the arrangement of the hcIg is the same as that of a normal antibody.

IgNAR is also a homodimer in which two heavy chains are covalently bound to each other by a disulfide bond, similarly to hcIg. Each heavy chain has a variable region at the N-terminus, and the variable region has three complementary determining regions (CDRs) that significantly contribute to the specific binding with an antigen. The three complementary determining regions are referred to as CDR1, CDR2, and CDR3 from the N-terminus. Since each heavy chain constituting the IgNAR can specifically bind to an antigen even though it is a monomer, one IgNAR has two regions in which an antigen can be bound in a molecule. In the variable region, a region other than the CDR is referred to as a framework region (FR), and the arrangement of the IgNAR is the same as that of a normal antibody.

The framework region and the CDR in the variable region of hcIg or IgNAR can be specified from a public DNA database including a reproductive cell lineage antibody gene sequence. For example, the reproductive cell lineage DNA sequence and amino acid sequence of the human heavy chain variable region gene can be selected from the "VBase" human reproductive cell line sequence database (available at www.mrccpe.cam.ac.uk/vbase on the Internet). In addition, the framework region and the CDR can also be specified from DNA sequences and amino acid sequences described in published documents, for example, "Kabat EA. Sequences of Proteins of Immunological Interest, 5th edition, US Department of Health and Human Services, NIH Publication No. 91-3242 (1991)", "Tomlinson IM. J. fol. Biol. 227. 776-98 (1992)", "Cox JPL. Eur. J Immunol. 24:827-836 (1994)", and the like. For example, since the variable region gene of the human heavy chain has homology with the variable region of hcIg, the framework region and the CDR of hcIg can also be specified based on information on the variable region gene of the human heavy chain. A method of specifying the framework region and the CDR is not particularly limited.

However, not limited to this, in each region specified as the FR region, (1) a region including 1 to 5 amino acids from the N-terminus and/or 1 to 5 amino acids at the C-terminus, (2) a region including 1 to 3 amino acids from the N-terminus and/or 1 to 3 amino acids at the C-terminus, or (3) a region including 1 or 2 amino acids from the N-terminus and/or 1 or 2 amino acids at the C-terminus can be included in the CDR region without being included in the FR region. In general, even a region which is regarded as FR can be included in the CDR as long as it is considered to be involved in the structural maintenance of the CDR or the binding to the antigen and to have a function of substantially determining the complementarity of the antibody. On the contrary, in each region specified as the CDR region, (1) a region including 1 to 5 amino acids from the N-terminus and/or 1 to 5 amino acids at the C-terminus, (2) a region including 1 to 3 amino acids from the N-terminus and/or 1 to 3 amino acids at the C-terminus, or (3) a region including 1 or 2 amino acids from the N-terminus and/or 1 or 2 amino acids at the C-terminus can also be included in the FR region without being included in the CDR region. In general, even a region which is regarded as CDR can be included in the FR in the present invention as long as the region is not involved in maintaining the structure of the CDR or in binding to an antigen and does not substantially have a function of determining the complementarity of the antibody.

A constant region is present at the C-terminus of the variable region of the heavy chain antibody via a hinge region. A constant region is also present at the C-terminus of the variable region of IgNAR. These constant regions hardly contribute to the specific binding of the heavy chain antibody to the antigen.

The heavy chain antibody is a homodimer covalently bound in a hinge region by a disulfide bond. However, the heavy chain antibody does not need to be a homodimer in order to bind to the antigen, and each heavy chain constituting the heavy chain antibody can be bound to the antigen alone.

The monomer of the heavy chain constituting the heavy chain antibody can be obtained by deleting a cysteine residue forming a disulfide bond in the amino acid sequence of the hinge region of the heavy chain or by substituting the cysteine residue with another amino acid. Such a monomer of a heavy chain can be produced as a recombinant protein using a gene recombinant technique. In the present specification, such heavy chain is referred to as a "single heavy chain antibody". The single heavy chain antibody is included in the heavy chain antibody as long as it can bind to the antigen of the original heavy chain antibody. The single heavy chain antibody can be an antibody in which the single heavy chain antibodies are linked in series. For example, in the single heavy chain antibody, the C-terminus of one heavy chain antibody is bound to the N-terminus of another heavy chain antibody directly or via a peptide linker. The number of single heavy chain antibodies to be linked is 2 to 10, for example, 2. As the sequence of the peptide linker, the sequence described in the present specification can be adopted. Such antibody in which a plurality of single heavy chain antibodies are linked to each other is also included in the single heavy chain antibody.

A variable region of each heavy chain constituting the heavy chain antibody can bind to an antigen even in a case where a hinge region or/and a constant region is absent. A protein consisting of such a variable region can be produced as a recombinant protein, for example, by constructing a gene encoding only the amino acid sequence of the variable region of the heavy chain of a heavy chain antibody, incorporating the gene into an expression vector, and transfecting the vector into a host cell.

In the present specification, for convenience, the protein including a part or all of a variable region of a heavy chain constituting the heavy chain antibody and having an affinity for an antigen is considered to be included in the heavy chain antibody. In addition to the protein including a part or all of the variable region of the heavy chain constituting the heavy chain antibody, the protein including (1) a part or all of a hinge region or (2) a part of a constant region of the heavy chain in addition to a hinge region is also included. The heavy chain antibody is derived from hcIg or derived from IgNAR. A monomer of such a heavy chain antibody is also included in the single heavy chain antibody.

The heavy chain antibody derived from hcIg includes (1h) an antibody consisting of all or a part of a variable region of a heavy chain, (2h) an antibody consisting of all or a part of the variable region and a part of a hinge region of the heavy chain, (3h) an antibody consisting of all of the variable region and a hinge region of the heavy chain, and (4h) an antibody including a part of a constant region of the heavy chain in addition to a variable region and a hinge region of a heavy chain. Here, a part of the constant region included in the heavy chain antibody is, for example, a portion corresponding to a part or all of C_{H}2 of the heavy chain. The heavy chain antibody derived from IgNAR includes (1I) an antibody consisting of all or a part of a variable region of a heavy chain, (2I) an antibody consisting of the variable region and a part of a hinge region of the heavy chain, (3I) an antibody consisting of all of the variable region and a hinge region of the heavy chain, and (4I) an antibody including a part of a constant region of the heavy chain in addition to a variable region and a hinge region of a heavy chain. Here, a part of the constant region included in the heavy chain antibody is, for example, a portion corresponding to a part or all of C_{H}2 of the heavy chain. In the present specification, the proteins (1h) to (4h) including the variable region of a heavy chain antibody derived from hcIg, and the proteins (II) to (4I) including the variable region of a heavy chain antibody derived from IgNAR are collectively referred to as heavy chain antibody variable domain peptide (heavy chain antibody variable region peptide). Therefore, the heavy chain antibody variable region peptide includes a peptide in which two peptide chains are bound to each other by a disulfide bond and two antigen binding sites are present, and a peptide consisting of one peptide chain in which one antigen binding site is present.

In the present specification, the heavy chain antibody recognizes a molecule present on the surface of a vascular endothelial cell, particularly a brain vascular endothelial cell, as an antigen. The species of the vascular endothelial cell and the brain vascular endothelial cell is not particularly limited, but is preferably a human cell. The molecule present on the surface of these cells and to be recognized as an antigen by the heavy chain antibody is preferably a transferrin receptor (TfR), an insulin receptor (IR), a leptin receptor, a lipoprotein receptor, an IGF receptor, OATP-F, an organic anion transporter, MCT-8, or a monocarboxylate transporter, more preferably TfR or an insulin receptor, and still more preferably TfR.

The heavy chain antibody variable region peptide having an affinity for a molecule present on the surface of a vascular endothelial cell, particularly a brain vascular endothelial cell is referred to as a blood brain barrier heavy chain antibody variable region peptide (BBB heavy chain antibody variable region peptide). In addition, the BBB heavy chain antibody variable region peptide and all peptides derived from the BBB heavy chain antibody variable region peptide, which have an affinity for a molecule present on the surface of a vascular endothelial cell, are referred to as a BBB affinity peptide. In the present specification, the protein of above-described (1h) or (2h), which includes a variable region of a heavy chain antibody derived from hcIg and consists of one peptide chain, is referred to as an immunoglobulin single variable domain peptide (immunoglobulin single variable region peptide). The immunoglobulin single variable domain peptide contained in the BBB heavy chain antibody variable region peptide can also be particularly referred to as a BBB immunoglobulin single variable domain peptide. In the present invention, VHH, a VHH domain, a VHH antibody, and Nanobody (trademark of Ablynx N.V.) are included in the immunoglobulin single variable domain peptide and are used interchangeably depending on the context. Therefore, for example, the VHH having an affinity for TfR is included in the BBB immunoglobulin single variable domain peptide.

The heavy chain antibody variable region peptide having an affinity for anti-hTfR is referred to as an anti-hTfR heavy chain antibody variable region peptide. In addition, all of the peptides having an affinity for anti-hTfR, which are derived from the anti-hTfR heavy chain antibody variable region peptide and the anti-hTfR heavy chain antibody variable region peptide, are referred to as an hTfR affinity peptide. The VHH having an affinity for hTfR is included in particular in the hTfR affinity peptide. The same applies to the VHH having an affinity for the other molecules present on the surface of the cells, and for example, the VHH having an affinity for hIR is included in the hIR affinity peptide. The number of amino acids constituting the BBB affinity peptide is not particularly limited, but usually 80 to 200, preferably 80 to 150, more preferably 100 to 130, and still more preferably 110 to 125. For example, the BBB affinity peptide is composed of 113, 115, 119, or 120 amino acids. The term "peptide" in the present specification is synonymous with "polypeptide" or "protein", and can be appropriately replaced with each other. Hereinafter, the characteristics or the like of the heavy chain antibody variable region peptide having an affinity for a human transferrin receptor (hTfR) will be described in detail as an example, but the description thereof can be generally applied to the heavy chain antibody variable region peptide having an affinity for another molecule present on the surface of the cells.

The antibody usually consists of a light chain and a heavy chain, whereas the VHH consists of one heavy chain. Therefore, a step of manufacturing a fusion protein in which the VHH, as the hTfR affinity peptide, is bound to a different protein (A) can be simplified. For example, in a case where a normal antibody is used as the hTfR affinity peptide, it is necessary to express two proteins, but in a case of the VHH, it is sufficient to express one protein.

The heavy chain antibody variable region peptide having an affinity for a human transferrin receptor is particularly referred to as an anti-hTfR heavy chain antibody variable region peptide. In the present specification, the protein of above-described (1h) or (2h), which includes a variable region of a heavy chain antibody derived from hcIg and consists of one peptide chain, is referred to as an immunoglobulin single variable domain peptide. The immunoglobulin single variable domain peptide having an affinity for a human transferrin receptor (hTfR) is particularly an hTfR immunoglobulin single variable domain peptide or a human transferrin receptor affinity peptide (hTfR affinity peptide). The hTfR affinity peptide is a peptide composed of preferably 100 to 130 amino acids and more preferably 110 to 125 amino acids. For example, the hTfR affinity peptide is composed of 113, 115, or 119 amino acids. In addition, the VHH, the VHH domain, the VHH antibody, and Nanobody having an affinity for hTfR are each referred to as hTfR-VHH, hTfR-VHH domain, hTfR-VHH antibody, and hTfR-Nanobody.

The hTfR affinity peptide also includes, in addition to a peptide of the types described as above-described (1h) to (4h) and (1I) to (4I), a peptide in which mutation such as substitution, deletion, or addition is applied thereto, as long as the peptide has an affinity for hTfR. In addition, the hTfR immunoglobulin single variable domain peptide also includes, in addition to a peptide of the types described as above-described (1h) or (2h), a peptide in which mutation such as substitution, deletion, or addition is applied thereto, as long as the peptide has an affinity for hTfR. The mutation of the hTfR immunoglobulin single variable domain peptide will be described in detail below.

In the present invention, the term "human transferrin receptor" or "hTfR" refers to a membrane protein having the amino acid sequence set forth in SEQ ID NO: 1. The anti-hTfR antibody of the present invention, in one embodiment of the present invention, specifically binds to a portion from the cysteine residue at 89th position from the N-terminus to the phenylalanine at the C-terminus in the amino acid sequence set forth in SEQ ID NO: 1 (extracellular region of hTfR), but the present invention is not limited thereto. In addition, in the present invention, the term "monkey transferrin receptor" or "monkey TfR" particularly refers to a membrane protein having the amino acid sequence set forth in SEQ ID NO: 2 derived from cynomolgus monkey (Macaca fascicularis). In addition, in the present invention, the term "mouse transferrin receptor" or "mouse TfR" particularly refers to a membrane protein having an amino acid sequence set forth in SEQ ID NO: 3 derived from a mouse. The anti-hTfR antibody of the present invention, in one embodiment of the present invention, binds to a portion from the cysteine residue at 89th portion from the N-terminus to the phenylalanine at the C-terminus in the amino acid sequence set forth in SEQ ID NO: 2 (extracellular region of monkey TfR) and/or a portion from the cysteine residue at 89th portion from the N-terminus to the phenylalanine at the C-terminus in the amino acid sequence set forth in SEQ ID NO: 3 (extracellular region of mouse TfR), but the present invention is not limited thereto.

A plurality of hTfR affinity peptides can also be bound directly or via a peptide linker. In this case, the plurality of hTfR affinity peptides to be bound to each other may all have the same amino acid sequence, or may have different amino acid sequences. The number of the hTfR affinity peptides to be bound is not particularly limited, but is, for example, 2 to 10, 2 to 5, 2, 3, or the like. By binding a plurality of hTfR affinity peptides, it is possible to impart properties such as improvement of the affinity for anti-hTfR. In the present specification, a peptide in which a plurality of hTfR affinity peptides are linked in this manner is referred to as a tandem type hTfR affinity peptide. The tandem type hTfR affinity peptide is included in the hTfR affinity peptide. The same can be said for the immunoglobulin single variable domain peptide and the anti-hTfR heavy chain antibody variable region peptide.

The hTfR affinity peptide can also be obtained as a recombinant protein by the same method.
(1a) A recombinant human transferrin receptor (rhTfR) is produced using a cell to which an expression vector in which the hTfR gene has been incorporated has been transferred.
(2a) Camelidae animals capable of producing hcIg or fishes capable of producing IgNAR, particularly cartilaginous fishes, such as a shark, are immunized with rhTfR.
(3a) Antibody-producing cells are taken out from the immunized living thing using rhTfR. Here, since the antibody-producing cells are present in particular in peripheral blood, bone marrow, a spleen, and the like, cells in the peripheral blood, bone marrow-derived cells, spleen cells, and the like are collected as the antibody-producing cells.
(4a) cDNA is synthesized from mRNA extracted from the antibody-producing cell by a reverse transcription reaction.
(5a) PCR reaction is carried out using the cDNA as a template to amplify a DNA fragment containing a gene encoding a variable region of a heavy chain antibody.
(6a) A DNA fragment encoding a variable region of a heavy chain antibody is incorporated into an expression vector, and a host cell is transformed with the expression vector to obtain expressing cells of the variable region of the heavy chain antibody.
(7a) From the expressing cells of the variable region of the heavy chain antibody, a cell expressing the variable region of the heavy chain antibody that recognizes hTfR (expressing cell of the variable region of the anti-hTfR heavy chain antibody) is selected. A gene encoding a variable region of a heavy chain antibody that recognizes hTfR can also be amplified by performing a PCR reaction and isolated from the cell selected here.
(8a) The expressing cell of the variable region of the anti-hTfR heavy chain antibody is cultured, and the recombinant anti-hTfR heavy chain antibody variable region peptide is expressed in the culture medium or in the cell.
(9a) The expressed recombinant anti-hTfR heavy chain antibody variable region peptide is purified.

The expressing cell of the variable region of the heavy chain antibody obtained in Step (6a) of acquiring the anti-hTfR heavy chain antibody that recognizes the above-described hTfR does not necessarily express the variable region of the heavy chain antibody that recognizes hTfR. Therefore, a step of selecting a cell that produces a variable region of a heavy chain antibody having a desired characteristic (affinity for hTfR) from the expressing cell of the variable region of the heavy chain antibody obtained in Step (6a), that is, Step (7a) is required. As a method for selecting a cell that produces the anti-hTfR heavy chain antibody variable region peptide, a method described below is effective.

The expressing cell of the variable region of the heavy chain antibody obtained in Step (6a) is seeded in a 96-well plate such that one cell is generally included in one well, and cultured, and then a culture supernatant is collected from each well. The recombinant hTfR is added to a plate and held thereon, and then the collected culture supernatant is added thereto, whereby the variable region of the anti-hTfR heavy chain antibody contained in the culture supernatant is bound to the recombinant hTfR on the plate. Next, the plate is washed after removing the culture supernatant from the plate to remove the antibody that is not bound to the recombinant hTfR. Next, the amount of the antibody held on the plate is measured. According to this method, the higher the affinity of the antibody contained in the culture supernatant of the antibody-producing cell added to the plate for hTfR is, the larger the amount of the antibody held in the plate is. Therefore, the amount of the antibody held on the plate is measured, and a cell corresponding to the plate on which a larger amount of the antibody is held can be selected as a cell line that produces an anti-hTfR heavy chain antibody variable region peptide having a relatively high affinity for hTfR. The mRNA extracted from the cell line selected in this manner is reverse-transcribed to obtain cDNA, and a PCR reaction is carried out using the cDNA as a template, whereby a DNA fragment containing a gene encoding the anti-hTfR heavy chain antibody variable region peptide can be amplified and isolated.

As another method of obtaining the anti-hTfR heavy chain antibody as a recombinant protein, there is a method including the following steps. This method includes a step of presenting the variable region of the heavy chain antibody encoded by the gene encoding the variable region of the heavy chain antibody on a phage to select a phage presenting the variable region of the heavy chain antibody having a desired property. Such step is called a phage display method, and is a well-known technique described in international publications of unexamined applications (WO1997/09436A and WO1995/11317A). The method of obtaining the anti-hTfR heavy chain antibody that recognizes hTfR as a recombinant protein by applying the phage display method includes, for example, Steps (1b) to (9b). The hTfR affinity peptide can also be obtained as a recombinant protein by the same method.
(1b) A recombinant human transferrin receptor (rhTfR) is produced using a cell to which an expression vector in which the hTfR gene has been incorporated has been transferred.
(2b) Camelidae animals capable of producing hcIg or fishes capable of producing IgNAR, particularly cartilaginous fishes, such as a shark, are immunized with rhTfR.
(3b) Antibody-producing cells are taken out from the immunized living thing using rhTfR. Here, since the antibody-producing cells are present in particular in peripheral blood, bone marrow, a spleen, and the like, cells in the peripheral blood, bone marrow-derived cells, spleen cells, and the like are collected as the antibody-producing cells.
(4b) cDNA is synthesized from mRNA extracted from the antibody-producing cell by a reverse transcription reaction.
(5b) PCR reaction is carried out using the cDNA as a template to amplify a DNA fragment containing a gene encoding a variable region of a heavy chain antibody.
(6b) A DNA fragment encoding a variable region of a heavy chain antibody is incorporated into a phagemid, the phagemid is transfected to a host cell, and then the host cell is cultured to release a phage having the variable region of a heavy chain antibody on a capsid surface into a medium.
(7b) A solution containing the collected phage is applied onto a column filled with a carrier holding rhTfR, and a phage having a variable region of a heavy chain antibody, which has an affinity for rhTfR, on a capsid surface is bound to the column. After washing the column, the phage bound to hTfR is eluted from the column. Alternatively, a solution containing the collected phage is added to a plate holding rhTfR, and a phage having a variable region of a heavy chain antibody, which has an affinity for rhTfR, on a capsid surface is bound to the plate.
(8b) Only the phages that present a variable region of a heavy chain antibody that recognizes hTfR, which is held on a column or a plate, are collected, and a phagemid contained in the phage is used as a template to amplify a gene encoding the variable region of the heavy chain antibody that recognizes hTfR by a PCR reaction.
(9b) The amplified gene is incorporated into an expression vector, and a host cell is transformed with the expression vector to obtain expressing cells of the variable region of the heavy chain antibody that recognizes hTfR.
(10b) The expressing cell of the anti-hTfR heavy chain antibody variable region peptide is cultured, and the recombinant anti-hTfR heavy chain antibody variable region peptide is expressed in the culture medium or in the cell.
(11b) The expressed recombinant anti-hTfR heavy chain antibody variable region peptide is purified.

In Steps (1b) to (3b) of the above-described phage display method, it is necessary to take out the antibody-producing cells from the living thing immunized with rhTfR. Alternatively, a pool of mRNA obtained from an antibody-producing cell of a living thing capable of expressing a heavy chain antibody or DNA fragments synthesized using the mRNA as a template can be prepared in advance, and the DNA fragments containing a gene encoding a variable region of a heavy chain antibody can also be amplified using the DNA fragment as a template. According to the phage display method, for example, 1 × 10¹⁰ phages can be screened at once, so that a phage that presents a variable region of a heavy chain antibody that recognizes hTfR even from cells of a non-immune animal may be selected. In this case, it is not necessary to immunize a living thing, and it is not necessary to take out an antibody-producing cell from the living thing.

Step (7b) is for selecting a phage that presents a variable region of a heavy chain antibody that recognizes rhTfR. Here, by adjusting the conditions for binding the phage to the column (or plate) and/or the conditions for washing the column (or plate), the affinity of the selected phage for rhTfR can be adjusted. In addition, by repeating Step (7b) while gradually making the condition more severe, a phage having higher affinity for rhTfR is selected each time the step is repeated, so that only a phage that presents a variable region of a heavy chain antibody having high affinity for rhTfR can be efficiently obtained. In addition, mutation may be incorporated into the genome in a process of replicating the phage genome. In this manner, the affinity of the variable region of the heavy chain antibody encoded in the phage genome for rhTfR is changed, so that by repeating Step (7), there is a possibility that an antibody having a higher affinity for rhTfR is obtained by randomly incorporating a mutation into the genome.

The step in a case where Step (7b) is repeated is, for example, a step shown in the following (7b').
(7b') A culture medium containing the collected phages is applied onto a column filled with a carrier holding rhTfR, and a phage that presents a variable region of a heavy chain antibody that recognizes rhTfR is bound to the column. Alternatively, a solution containing the collected phage is added to a plate holding rhTfR, and a phage having a variable region of a heavy chain antibody, which has an affinity for rhTfR, on a capsid surface is bound to the plate. After washing the column or the plate, a phage that presents a variable region of a heavy chain antibody that recognizes hTfR is eluted from the column or the plate. The eluted phage is allowed to infect host cells, and then the host cells are cultured, and the phage that presents a variable region of a heavy chain antibody on a surface is collected in the culture medium. The culture medium containing the collected phage is applied on a column chromatography filled with a carrier holding rhTfR, or is added to a plate holding rhTfR. In Step (7b'), the phage genome can also be actively mutated by bringing the phage into contact with a mutagen or adding a mutagen to a culture medium of a host cell infected with the phage. By irradiating the phage or the host cell infected with the phage with electromagnetic waves such as ultraviolet rays and gamma rays, a mutation can also be actively introduced into the phage genome.

Any steps of Step (1a) to (9a) and Steps (1b) to (11b) can also include a step of isolating a gene encoding the variable region of the anti-hTfR heavy chain antibody. It is also possible to translate the nucleotide sequence of the gene encoding the isolated anti-hTfR heavy chain antibody into an amino acid sequence including the variable region of the anti-hTfR heavy chain antibody, and to artificially synthesize a DNA fragment encoding this amino acid sequence. In a case where the DNA fragment is artificially synthesized, the DNA fragment can be synthesized by selecting a suitable codon for expressing a gene in a host cell. By transferring an expression vector in which such a DNA fragment has been incorporated into a host cell, the expression level of the anti-hTfR heavy chain antibody variable region peptide in the host cell can be increased. The expression level of the rhTfR affinity peptide can also be increased in the same manner.

In addition, the cell used as the host cell in the above-described step is not particularly limited as long as the cell is a cell that can express a peptide including the variable region of a heavy chain antibody by transferring an expression vector in which a gene encoding the variable region of a heavy chain antibody is incorporated, regardless of whether the cell is a prokaryotic cell or a eukaryotic cell, and particularly, Escherichia coli, CHO cells derived from Chinese hamster ovary, or NS/0 cells derived from mouse myeloma is preferable. In addition, as the expression vector used for incorporating and expressing a gene encoding the variable region of a heavy chain antibody, an expression vector can be used without particular limitation as long as the expression vector can express a peptide including the variable region of the heavy chain antibody in a case of being transferred into a host cell. The gene incorporated into the expression vector is arranged downstream of a DNA sequence capable of regulating the frequency of transcription of the gene in the host cell (gene expression control site). Examples of the gene expression control site that can be used in the present invention include a promoter derived from cytomegalovirus, an SV40 early promoter, a human elongation factor-1α (EF-1α) promoter, a human ubiquitin C promoter, and the like.

The amino acid sequence of the variable region in the anti-hTfR heavy chain antibody variable region peptide obtained by the above-described step includes an amino acid sequence of four FRs and three CDRs, that is, FR1, CDR1, FR2, CDR2, FR3, CDR3, and FR4, basically from the N-terminus. However, the present invention is not limited thereto, and as long as it can specifically bind to hTfR, the amino acid sequence of the variable region may be an amino acid sequence in which a part or all of FR1 is deleted, an amino acid sequence in which a part or all of FR4 is deleted, or an amino acid sequence in which a part or all of FR1 is deleted and a part or all of FR4 is deleted. The amino acid sequences in which a part of the FR region is deleted can be included in the variable region of the anti-hTfR heavy chain antibody. The same applies to the hTfR affinity peptide.

Suitable examples of the anti-hTfR heavy chain antibody variable region peptide in one embodiment of the present invention include a peptide including amino acid sequences of an hTfR affinity peptide 1 to 3. hTfR affinity peptides 1 to 3 each include an amino acid sequence set forth in SEQ ID NOs: 4 to 6. The amino acid sequences set forth in SEQ ID NOs: 4, 5, and 6 are all amino acid sequences of a variable region of a heavy chain antibody. All of the hTfR affinity peptides 1 to 3 include an amino acid sequence of four FRs and three CDRs, that is, FR1, CDR1, FR2, CDR2, FR3, CDR3, and FR4 from the N-terminus. All of the hTfR affinity peptides 1 to 6 are included in the hTfR affinity peptide.

The CDR1 of the hTfR affinity peptide 1 is a region including the amino acid sequence set forth in SEQ ID NO: 7 or 8, the CDR2 thereof is a region including the amino acid sequence set forth in SEQ ID NO: 9 or 10, and the CDR3 thereof is a region including the amino acid sequence set forth in SEQ ID NO: 11 or 12. In the hTfR affinity peptide 1, each of the amino acid sequences of CDRs can be combined arbitrarily. For example, the amino acid sequences may be combined such that the CDR1 is a region including the amino acid sequence set forth in SEQ ID NO: 7, the CDR2 is a region including the amino acid sequence set forth in SEQ ID NO: 9, and the CDR3 is a region including the amino acid sequence set forth in SEQ ID NO: 11, or the CDR1 is a region including the amino acid sequence set forth in SEQ ID NO: 8, the CDR2 is a region including the amino acid sequence set forth in SEQ ID NO: 10, and the CDR3 is a region including the amino acid sequence set forth in SEQ ID NO: 12. In addition, CDR2 may further include an amino acid sequence of VKG at the C-terminus of the amino acid sequence set forth in SEQ ID NO: 10. In addition, any CDR may consist of these amino acid sequences.

The CDR1 of the hTfR affinity peptide 2 is a region including the amino acid sequence set forth in SEQ ID NO: 7 or 8, the CDR2 thereof is a region including the amino acid sequence set forth in SEQ ID NO: 9 or 10, and the CDR3 thereof is a region including the amino acid sequence set forth in SEQ ID NO: 11 or 12. In the hTfR affinity peptide 2, each of the amino acid sequences of CDRs can be combined arbitrarily. For example, the amino acid sequences may be combined such that the CDR1 is a region including the amino acid sequence set forth in SEQ ID NO: 7, the CDR2 is a region including the amino acid sequence set forth in SEQ ID NO: 9, and the CDR3 is a region including the amino acid sequence set forth in SEQ ID NO: 11, or the CDR1 is a region including the amino acid sequence set forth in SEQ ID NO: 8, the CDR2 is a region including the amino acid sequence set forth in SEQ ID NO: 10, and the CDR3 is a region including the amino acid sequence set forth in SEQ ID NO: 12. In addition, CDR2 may further include an amino acid sequence of VKG at the C-terminus of the amino acid sequence set forth in SEQ ID NO: 10. In addition, any CDR may consist of these amino acid sequences.

The CDR1 of the hTfR affinity peptide 3 is a region including the amino acid sequence set forth in SEQ ID NO: 13 or 14, the CDR2 thereof is a region including the amino acid sequence set forth in SEQ ID NO: 15 or 16, and the CDR3 thereof is a region including the amino acid sequence set forth in SEQ ID NO: 17 or 18. In the hTfR affinity peptide 3, each of the amino acid sequences of CDRs can be combined arbitrarily. For example, the amino acid sequences may be combined such that the CDR1 is a region including the amino acid sequence set forth in SEQ ID NO: 13, the CDR2 is a region including the amino acid sequence set forth in SEQ ID NO: 15, and the CDR3 is a region including the amino acid sequence set forth in SEQ ID NO: 17, or the CDR1 is a region including the amino acid sequence set forth in SEQ ID NO: 14, the CDR2 is a region including the amino acid sequence set forth in SEQ ID NO: 16, and the CDR3 is a region including the amino acid sequence set forth in SEQ ID NO: 18. In addition, CDR2 may further include an amino acid sequence of VKG at the C-terminus of the amino acid sequence set forth in SEQ ID NO: 16. In addition, any CDR may consist of these amino acid sequences.

In addition, the amino acid sequence of the variable region of the above-described hTfR affinity peptide may be an amino acid sequence in which a mutation is added as long as the peptide can specifically bind to hTfR. In a case where such mutation is applied, the amino acid sequence of the variable region of the hTfR affinity peptide after applying the mutation has preferably 80% or more identity, more preferably 85% or more identity, still more preferably 90% or more identity, even still more preferably 95% or more identity, and for example, 98% or more identity to the original amino acid sequence.

In addition, in a case of where mutation is applied to the amino acid sequence of the variable region, mutation may not be applied to the amino acid sequences of CDR1, CDR2, and CDR3, and mutation may be applied only to the FR region. In a case where such mutation is applied, the amino acid sequence of the variable region after the mutation is applied has preferably 85% or more identity, more preferably 90% or more identity, still more preferably 95% or more identity, and for example, 98% or more identity to the original amino acid sequence. In addition, in a case of where mutation is applied to the amino acid sequence of the variable region, mutation may not be applied to the amino acid sequences of FR region, and mutation may be applied only to the CDR region. In a case where such mutation is applied, the amino acid sequence of the variable region after the mutation is applied has preferably 90% or more identity, more preferably 95% or more identity, and for example, 98% or more identity to the original amino acid sequence. In addition, the mutation can be applied to both the FR region and the CDR region.

The identity between the amino acid sequence of the original variable region and the amino acid sequence of the variable region to which the mutation is applied can be easily calculated using a known homology calculation algorithm. Examples of such an algorithm include BLAST (Altschul SF. J Mol. Biol. 215. 403-10, (1990)), a similarity search method of Pearson and Lipman (Proc. Natl. Acad. Sci. USA. 85. 2444 (1988)), a local homology algorithm of Smith and Waterman (Adv. Appl. Math. 2. 482-9 (1981)), and the like. In addition, in the entire present specification, the identity refers to the identity calculated by these algorithms. In the present specification, the terms "homology of amino acid sequence" and "identity of amino acid sequence" are used interchangeably.

The case where the mutation such as substitution, deletion, and addition are applied to the amino acid sequence of the variable region of the hTfR affinity peptide will be described in detail below.

In a case where amino acids of the amino acid sequence of the variable region of the hTfR affinity peptide are substituted with other amino acids, the number of the amino acids to be substituted is preferably 1 to 20, more preferably 1 to 15, still more preferably 1 to 10, and even still more preferably 1 to 5, and for example, 1, 2, or 3. In a case where amino acids in the amino acid sequence of the variable region are deleted, the number of the amino acids to be deleted is preferably 1 to 20, more preferably 1 to 15, still more preferably 1 to 10, and even still more preferably 1 to 5, and for example, 1, 2, or 3. In addition, mutations in which the substitution and the deletion of these amino acids are combined can also be applied. In a case where an amino acid is added to the variable region, preferably 1 to 20, more preferably 1 to 15, still more preferably 1 to 10, and even still more preferably 1 to 5 amino acids, for example, 1, 2, or 3 amino acids are added into the amino acid sequence or to the N-terminus or the C-terminus of the amino acid sequence of the variable region. Mutations in which addition, substitution, and deletion in these amino acids are combined can also be applied.

In a case where the mutation such as substitution, deletion, and addition is applied to the amino acid sequence of the variable region of the hTfR affinity peptide, the mutation can be applied only to the FR region without applying the mutation to the amino acid sequence of the CDR region (CDR1, CDR2, and CDR3). The number of the amino acids to be substituted in a case where the substitution is added only to the FR region is preferably 1 to 12, more preferably 1 to 10, still more preferably 1 to 8, and even still more preferably 1 to 4, and for example, 1, 2, or 3. In addition, in a case where the amino acid is deleted only in the amino acid sequence of the FR region, the number of the amino acids to be deleted is preferably 1 to 8, more preferably 1 to 4, still more preferably 1 to 3, and even still more preferably 1 to 2, and for example, 1 or 2. In addition, mutations in which the substitution and the deletion of these amino acids are combined can also be applied. In addition, in a case where the amino acid is added only in the amino acid sequence of the FR region, preferably 1 to 8, more preferably 1 to 4, still more preferably 1 to 3, and even still more preferably 1 to 2 amino acids, for example, 1 or 2 amino acids are added in the amino acid sequence or to the N-terminus or the C-terminus of the variable region. Mutations in which addition, substitution, and deletion in these amino acids are combined can also be applied.

In a case where the mutation is applied only to the FR region, a method of substituting an amino acid residue of the FR region of the hTfR affinity peptide with a corresponding amino acid residue of the FR region in the variable region of the IgG type human antibody is known. In the present specification, this method is referred to as humanization of the hTfR affinity peptide. Such method is disclosed, for example, in Vincle C., et. Al., J biol Chem. 284. 3273-84 (2009). In a case where the original antibody is an antibody of an alpaca, the antibody may be recognized as an antigen in a case of being administered to a human. It is expected that the humanized antibody has lower antigenicity than the original antibody. In a case where the mutation is applied only to the FR region, the humanization is one of preferable embodiments. In the present specification, the hTfR affinity peptide also includes a humanized hTfR affinity peptide. The humanized hTfR affinity peptide also includes the humanized VHH in a case where the hTfR affinity peptide is a VHH.

In a case where the mutation such as substitution, deletion, and addition is applied to the amino acid sequence of the variable region of the hTfR affinity peptide, the mutation can be applied only to the CDR region without applying the mutation to the amino acid sequence of the FR region. The number of the amino acids to be substituted in a case where the substitution is applied only to the CDR region is preferably 1 to 4, more preferably 1 to 3, and still more preferably 1 or 2, and for example, 1 or 2. In addition, in a case where the amino acid is deleted only in the amino acid sequence of the CDR region, the number of the amino acids to be deleted is preferably 1 to 4, more preferably 1 to 3, and still more preferably 1 or 2, and for example, 1 or 2. In addition, mutations in which the substitution and the deletion of these amino acids are combined can also be applied. In addition, in a case where the amino acid is added only to the amino acid sequence of the FR region, preferably 1 to 4, more preferably 1 to 3, and still more preferably 1 or 2 amino acids, for example, 1 or 2 amino acids are added into the amino acid sequence or to the N-terminus or the C-terminus of the variable region. Mutations in which addition, substitution, and deletion in these amino acids are combined can also be applied.

In a case where the mutation such as substitution, deletion, and addition are applied to the amino acid sequence of the variable region of the hTfR affinity peptide, the above-described mutation in which the mutation in the FR region and the mutation in the CDR region are combined can also be applied to the amino acid sequence.

The above-described substitution of an amino acid in the amino acid sequence of the variable region with another amino acid occurs, for example, within an amino acid family related to the side chain and chemical properties of the amino acid. It is predicted that such a substitution within the amino acid family does not cause a significant change in the function of the anti-hTfR heavy chain antibody (that is, it is a conservative amino acid substitution). Examples of such amino acid family include:
(1) aspartic acid and glutamic acid, which are acidic amino acids,
(2) histidine, lysine, and arginine, which are basic amino acids,
(3) phenylalanine, tyrosine, and tryptophan, which are aromatic amino acids,
(4) serine and threonine, which are amino acids having a hydroxyl group (hydroxyamino acids),
(6) cysteine, serine, threonine, asparagine, and glutamine, which are neutral hydrophilic amino acids,
(7) glycine and proline, which are amino acids that affect the alignment of the peptide chain,
(8) asparagine and glutamine, which are amide-type amino acids (polar amino acids),
(9) alanine, leucine, isoleucine, and valine, which are aliphatic amino acids,
(10) alanine, glycine, serine, and threonine, which are amino acids having a small side chain,
(11) alanine and glycine, which are amino acids having the particularly small side chain,
(12) valine, leucine, and isoleucine, which are amino acids having a branched chain.

The above-described conservative amino acid substitution is also applied to a case where an amino acid in an amino acid sequence of another protein such as human serum albumin (HSA) is substituted with another amino acid.

In a case where an amino acid is added to the C-terminus or the N-terminus by applying a mutation to the variable region, and the added amino acid is located between the hTfR affinity peptide and adifferent protein (A) when the hTfR affinity peptide is fused with the different protein (A), the added amino acid is defined as a part of the hTfR affinity peptide. In the fusion protein of the hTfR affinity peptide and the protein (A), a linker located between the hTfR affinity peptide and the protein (A) will be described in detail later. In the present specification, the term "different protein (A)" refers to a protein which should be administered to a human in a form bound to the hTfR affinity peptide and which should exhibit a physiological activity in the body of the human, and refers to a protein different from the hTfR affinity peptide.

The hTfR affinity peptide selected by the above-described step or the like has a certain affinity for hTfR. The variable region of the hTfR affinity peptide can be modified by introducing a mutation such as substitution, deletion, or addition into the amino acid sequence thereof so that the variable region of the anti-hTfR heavy chain antibody has desired characteristics. The introduction of the mutation into the amino acid sequence of the variable region of the anti-hTfR heavy chain antibody is carried out by applying a mutation to a gene corresponding to the amino acid sequence.

The affinity of the variable region of the hTfR affinity peptide for hTfR can be appropriately adjusted by applying a mutation such as substitution, deletion, or addition to the amino acid sequence constituting the variable region of the hTfR affinity peptide. For example, in a case where the affinity between the hTfR affinity peptide and the antigen is high and the dissociation constant in an aqueous solution is remarkably low, when the hTfR affinity peptide is administered into a living body, the variable region may not be dissociated from the antigen, and as a result, there is a possibility that a functional inconvenience may occur. In such a case, by introducing a mutation into the variable region, the dissociation constant can be adjusted in a stepwise manner to be 2 to 5 times, 5 to 10 times, 10 to 100 times, or the like of the original variable region, and the most preferable hTfR affinity peptide that matches the purpose can be acquired. On the contrary, by introducing the mutation, the dissociation constant can also be adjusted in a stepwise manner to 1/2 to 1/5 times, 1/5 to 1/10 times, 1/10 to 1/100 times, or the like of the original variable region.

The binding affinity between the hTfR affinity peptide and the transferrin receptor, that is, the dissociation constant can be measured by a method, for example, using a bio-layer interferometry (BLI) method, specifically, an Octet system. All or a part of transferrin receptors derived from an appropriate animal is fixed to a sensor, and a solution including a variable region of the hTfR affinity peptide is used as a sample to measure a binding rate constant (Kon) or a dissociation rate constant (Koff), whereby a dissociation constant can be calculated from the result. In addition, in the same manner, the dissociation constant can also be calculated by a method using a surface plasmon resonance (SPR) technique, specifically, a method using Biacore. For example, the measuring method described in Example 7 can be suitably used as a method of measuring the binding affinity between the hTfR affinity peptide and the transferrin receptor.

The introduction of the mutation such as substitution, deletion, and addition into the amino acid sequence of the variable region of the hTfR affinity peptide can be carried out, for example, by using a gene encoding the variable region of an anti-hTfR heavy chain antibody as a template and introducing a mutation at a specific site of the nucleotide sequence of the gene by a method such as PCR, or by introducing a random mutation.

Introduction of mutation into the amino acid sequence of the hTfR affinity peptide for the purpose of adjusting the affinity between the hTfR affinity peptide and hTfR can be carried out, for example, by incorporating a gene encoding the hTfR affinity peptide into a phagemid, producing a phage that expresses the hTfR affinity peptide on the capsid surface using the phagemid, and acting a mutagen or the like on the phage. The phage can be proliferated while introducing a mutation into a gene encoding the hTfR affinity peptide by a mutagen, and a phage expressing a single-stranded antibody having a desired dissociation constant can be selected from the proliferated phage by the above-described method.

In the hTfR affinity peptide having a relatively high affinity for hTfR, which is obtained by the above-described method, a dissociation constant (K_{D}) with hTfR, which is measured preferably by the method described in Example 7, is preferably 5 × 10⁻⁸ M or less and more preferably 2 × 10⁻⁸ M or less, and for example, 1 × 10⁻⁸ M or less, 5 × 10⁻⁹ M or less, and 1 × 10⁻⁹ M or less. Examples of the suitable peptide include a peptide in which a dissociation constant is 5 × 10⁻¹¹ M to 1 × 10⁻⁸ M, 2 × 10⁻¹¹ M to 1 × 10⁻⁸ M, 1 × 10⁻¹⁰ M to 1 × 10⁻⁸ M, or 1.0 × 10⁻⁹ M to 1.0 × 10⁻⁸ M.

By selecting the hTfR affinity peptide having an affinity for the monkey TfR from the hTfR affinity peptide obtained as described above, it is possible to obtain the hTfR affinity peptide having an affinity for both the human TfR and the monkey TfR. The hTfR affinity peptide having an affinity for the monkey TfR can be selected, for example, by an ELISA method using a recombinant monkey TfR produced by a gene recombination technique. In this ELISA method, the recombinant monkey TfR is immobilized on a plate, the hTfR affinity peptide is brought into contact with the plate, the unbound hTfR affinity peptide to the recombinant monkey TfR is removed from the plate, and the amount of the hTfR affinity peptide retained on the plate is measured. As the affinity for the recombinant monkey TfR is higher, the amount of the hTfR affinity peptide retained on the plate is larger, so that the hTfR affinity peptide corresponding to the plate on which more hTfR affinity peptides are retained can be selected as the hTfR affinity peptide having an affinity for the monkey TfR. The term "monkey" used herein preferably refers to a monkey other than a human, more preferably refers to a monkey classified into Cercopithecidae, still more preferably refers to a monkey classified into Macaca, and still more preferably refers to a monkey classified into Macaca, for example, a rhesus monkey or a cynomolgus monkey, and particularly refers to a cynomolgus monkey. The cynomolgus monkey is convenient for use in evaluating the drug efficacy of a drug applied using the hTfR affinity peptide. Similarly, it is possible to obtain a peptide having an affinity for TfR of both human and monkey by the same method for the variable region peptide of the anti-hTfR heavy chain antibody.

The hTfR affinity peptide having an affinity for hTfR of both human and monkey has an advantageous effect that the pharmacokinetics in the human body in a case of being administered can be observed using a monkey. For example, in a case where a pharmaceutical is developed by using the hTfR affinity peptide according to the embodiment of the present invention, by conducting a part of non-clinical tests such as a pharmacokinetic test of the pharmaceutical using monkeys, it is possible to more accurately predict the pharmacokinetics or the like in a case where the peptide is administered to a human. As a result, development of the pharmaceutical can be significantly promoted. The same can be applied to the variable region peptide of the anti-hTfR heavy chain antibody.

In the present invention, the hTfR affinity peptide having a relatively high affinity for hTfR and having an affinity for the TfR of a monkey is particularly as follows in a case of being measured by the method described in Example 7.
(a) the dissociation constants with hTfR and the monkey TfR are all 5 × 10⁻¹¹ M to 5 × 10⁻⁷ M, and
(b) the dissociation constants with hTfR and the monkey TfR are all 5 × 10⁻¹¹ M to 1 × 10⁻⁸ M,
(c) the dissociation constants with hTfR and the monkeyTfR are all 5 × 10⁻¹⁰ M to 1 × 10⁻⁸ M,
(d) the dissociation constants with hTfR and the monkey TfR are all 1 × 10⁻¹⁰ M to 5 × 10⁻⁷ M.

In particular, in (a) to (d), the ratio of the dissociation constant with hTfR to the dissociation constant with monkey TfR is preferably 1 to 10, for example, 3 to 10, 5 to 10, or 3 to 5 in a case where the value of the dissociation constant with hTfR is set to 1.

Since the dissociation constants of the hTfR affinity peptides 1 to 3 in the present embodiment with the TfR of the human and the monkey are any of (a) to (e) described above, it can be said that the behavior in the monkey body in a case of being administered to the monkey is approximated to the behavior in a case of being administered to the human. Therefore, in a drug applied with the hTfR affinity peptides 1 to 3, in a test using monkeys, a result reflecting the behavior in a case of being administered to a human is obtained. Therefore, as a result of the test using monkeys, more useful results can be obtained as a judgment material in a case of conducting a clinical trial using humans. In particular, since the ratio of the dissociation constant with hTfR to the dissociation constant with monkey TfR is in a range of 1 to 10 in a case where the value of the dissociation constant with hTfR is 1, the hTfR affinity peptides 1 and 2 are suitable for easily presuming the behavior in the human body from the behavior in the monkey body.

In a case where the hTfR affinity peptide in the embodiment of the present invention is administered to a living body by intravenous injection or the like, the hTfR affinity peptide can efficiently bind to hTfR present on endothelial cells of capillaries in the brain. The hTfR affinity peptide bound to hTfR is taken up into the brain by a mechanism such as endocytosis, exocytosis, and transcytosis, through the blood brain barrier. Therefore, by binding a protein, a low-molecular-weight compound, or the like to these hTfR affinity peptides, these substances can be efficiently passed through the blood brain barrier and reach the brain. In addition, the hTfR affinity peptide in the embodiment of the present invention passes through the blood brain barrier and then reaches the cerebral parenchyma of the cerebrum, the neuron-like cells of the hippocampus, the cerebellar parenchyma, or at least one of these. Therefore, by binding a drug that should act on these tissues or cells, specifically, a protein, a low-molecular-weight compound, a nucleic acid, or the like to the hTfR affinity peptide of the present invention, the drug can be made to reach these tissues or cells. Such a complex of the hTfR affinity peptide and the drug is referred to as an hTfR affinity peptide-drug complex in the present specification. The same can be said for the variable region peptide of the anti-hTfR heavy chain antibody and the immunoglobulin single variable domain peptide of hTfR (including VHH). Hereinafter, the hTfR affinity peptide will be described in detail, but the applications and the like are also applied to the anti-hTfR heavy chain antibody variable region peptide, the hTfR immunoglobulin single variable domain peptide, the hTfR-VHH, and the like.

In order to allow a substance (protein, low-molecular-weight compound, nucleic acid, or the like), which, in general, is not possible to pass through the blood brain barrier, and therefore can hardly or cannot exert a physiological or pharmacological action in the brain in the case of intravenous administration, to exert an action in the brain, it can be an effective means to form a complex with the hTfR affinity peptide accoring to the embodiment of the present invention. In particular, the hTfR affinity peptide of the present invention passes through the blood brain barrier and then reaches the cerebral cortex of the cerebrum, the neuron-like cells of the hippocampus, the cerebral cortex of the cerebellum, or at least any of these. Therefore, by intravenously administering or blood-administering these substances in a form of being bound to the hTfR affinity peptide of the present invention, it is possible to allow these substances to pass through the blood brain barrier and reach the central nervous tissue, and to exert or enhance the physiological activity thereof in the tissue or cell of the central nervous system. The same can be applied to the variable region peptide of the anti-hTfR heavy chain antibody.

As a method of binding the hTfR affinity peptide to such a substance (protein, low-molecular-weight compound, nucleic acid, or the like), there is a method of binding the hTfR affinity peptide through a non-peptide linker or a peptide linker. The same can be applied to the variable region peptide of the anti-hTfR heavy chain antibody. As the non-peptide linker, polyethylene glycol (PEG), polypropylene glycol, a copolymer of ethylene glycol and propylene glycol, a polyoxyethylated polyol, polyvinyl alcohol, polysaccharides, dextran, polyvinyl ether, a biodegradable polymer, a lipid polymer, chitins, hyaluronic acid, or a derivative thereof, or a combination thereof can be used. The peptide linker is a peptide chain or a derivative thereof, which is composed of 1 to 60 amino acids bound by a peptide bond, and in which the N-terminus and the C-terminus thereof form a covalent bond with any of a hTfR affinity peptide or protein, a low-molecular-weight compound, a nucleic acid, or the like, so that the anti-hTfR antibody and a protein, a low-molecular-weight compound, a nucleic acid, or the like are bound to each other.

A non-peptide linker in which the hTfR affinity peptide of the present invention and a desired different protein (A) are bound to each other by using PEG is particularly referred to as an hTfR affinity peptide-PEG-protein. The hTfR affinity peptide-PEG-protein can be produced by producing an hTfR affinity peptide-PEG by binding an hTfR affinity peptide and PEG, and then binding the hTfR affinity peptide-PEG and the different protein (A). Alternatively, the hTfR affinity peptide-PEG-protein can also be produced by producing a protein-PEG by binding the different protein (A) and PEG to each other, and then binding the protein-PEG and the hTfR affinity peptide to each other. In a case where PEG is bound to the hTfR affinity peptide and the different protein (A), PEG modified with a functional group such as a carbonate, a carbonylimidazole, an active ester of a carboxylic acid, an azlactone, a cyclic imidothione, an isocyanate, an isothiocyanate, an imidate, or an aldehyde is used. The functional group introduced into these PEGs is reacted with mainly the amino group in the hTfR affinity peptide and the different protein (A) in the molecule, whereby the PEG and the hTfR affinity peptide and the different protein (A) are covalently bound to each other. A molecular weight and a shape of the PEG used at this time are not particularly limited, and an average molecular weight (MW) thereof is preferably 500 to 60,000 and more preferably 500 to 20,000. For example, PEG having an average molecular weight of about 300, about 500, about 1,000, about 2,000, about 4,000, about 10,000, about 20,000, or the like can be suitably used as the non-peptide linker. The same applies to a case where the hTfR affinity peptide is bound to a desired low-molecular-weight compound, nucleic acid, or the like.

For example, the hTfR affinity peptide-PEG is obtained by mixing the hTfR affinity peptide and a polyethylene glycol (ALD-PEG-ALD) having an aldehyde group as a functional group such that the molar ratio of ALD-PEG-ALD to the antibody is 1:11, 1:12.5, 1:15, 1:110, 1:120, or the like, and adding a reducing agent such as NaCNBH₃ to the mixture to react. Next, the hTfR affinity peptide-PEG is reacted with the different protein (A) in the presence of a reducing agent such as NaCNBH₃, thereby obtaining an hTfR affinity peptide-PEG-protein. On the contrary, the hTfR affinity peptide-PEG-protein can also be obtained by first binding the different protein (A) and the ALD-PEG-ALD to produce a protein-PEG, and then binding the protein-PEG and the hTfR affinity peptide.

The hTfR affinity peptide and the different protein (A) can also be bound to each other by a peptide linker or directly to the C-terminus or the N-terminus of the hTfR affinity peptide, respectively, by a peptide bond. The fusion protein obtained by binding such an hTfR affinity peptide and a different protein (A) to each other can be obtained as a recombinant protein by incorporating a DNA fragment in which a cDNA encoding the different protein (A) is disposed in-frame with a 3'-terminal or 5'-terminal of a cDNA encoding the hTfR affinity peptide with a DNA fragment encoding an amino acid sequence of a peptide linker interposed therebetween into an expression vector for a eukaryotic cell such as a mammalian cell or a yeast or an expression vector for a prokaryotic cell such as Escherichia coli, and then culturing a host cell into which the expression vector has been introduced. In the present specification, the recombinant protein in which the hTfR affinity peptide and the different protein (A) are bound to each other is referred to as an hTfR affinity peptide-protein (A) fusion protein. Similarly, the recombinant protein in which the immunoglobulin single variable domain peptide is bound to the different protein (A) is referred to as an immunoglobulin single variable domain peptide-protein (A) fusion protein. In addition, a recombinant protein in which the heavy chain antibody variable region peptide is bound to a different protein (A) is referred to as a heavy chain antibody variable region peptide-protein (A) fusion protein. The hTfR affinity peptide-protein (A) fusion protein, the immunoglobulin single variable domain peptide-protein (A) fusion protein, and the heavy chain antibody variable region peptide-protein (A) fusion protein can be mutually replaced depending on the context in the present specification.

The peptide linker disposed between the hTfR affinity peptide and the different protein (A) is preferably a peptide chain consisting of 1 to 60 or 1 to 50, more preferably 1 to 17, still more preferably 1 to 10, and still more preferably 1 to 5 amino acids, but the number of amino acids constituting the peptide linker can be appropriately adjusted to 1, 2, 3, 1 to 17, 1 to 10, 10 to 40, 20 to 34, 23 to 31, 25 to 29, 27, or the like depending on the different protein (A) to be bound to the anti-hTfR antibody. Such a peptide linker is not limited to an amino acid sequence as long as the hTfR affinity peptide linked thereto has an affinity for hTfR and the different protein (A) linked by the peptide linker can exhibit a desired physiological activity under physiological conditions, but is preferably composed of glycine and serine.

Examples of a preferred peptide linker include a peptide linker consisting of one amino acid (for example, glycine or serine), the amino acid sequence Gly-Ser, the amino acid sequence Ser-Ser, the amino acid sequence Gly-Gly-Ser, the amino acid sequence set forth in SEQ ID NO: 19, the amino acid sequence set forth in SEQ ID NO: 20, the amino acid sequence set forth in SEQ ID NO: 21, the amino acid sequence set forth in SEQ ID NO: 22, the amino acid sequence set forth in SEQ ID NO: 23, the amino acid sequence set forth in SEQ ID NO: 24, and the amino acid sequence set forth in SEQ ID NO: 25. In addition, examples of the preferred peptide linker include a peptide linker consisting of one amino acid (for example, glycine or serine), a peptide linker including a sequence in which 2 to 10 or 2 to 5 amino acid sequences selected from the group consisting of the amino acid sequence Gly-Ser, the amino acid sequence Ser-Ser, the amino acid sequence Gly-Gly-Ser, the amino acid sequence of SEQ ID NO: 19, the amino acid sequence of SEQ ID NO: 20, and the amino acid sequence of SEQ ID NO: 21 are arranged in series. The peptide linker has a sequence consisting of 2 to 50 amino acids, a sequence consisting of 2 to 17 amino acids, 2 to 10 amino acids, 10 to 40 amino acids, 20 to 34 amino acids, 23 to 31 amino acids, 25 to 29 amino acids, or 27 amino acids. For example, a peptide linker including the amino acid sequence Gly-Ser can be suitably used as the peptide linker. In addition, a peptide linker including a total of 25 amino acids in which the amino acid sequence set forth in SEQ ID NO: 19 is repeated 5 times can also be suitably used as the peptide linker.

The hTfR affinity peptide can also be bound to an Fc region of human IgG (hIgG Fc region). By binding the hIgG Fc region to the hTfR affinity peptide, the stability of the hTfR affinity peptide in vivo, for example, the blood retention can be increased. Examples of such an hTfR affinity peptide to which the hIgG Fc region is bound include a peptide in which the hIgG Fc region is directly or via a peptide linker bound to the C-terminus of the hTfR affinity peptide, and a peptide in which the hIgG Fc region is directly or via a peptide linker bound to the N-terminus of the hTfR affinity peptide. The conjugate of the Fc region and the hTfR affinity peptide can be used to form a complex with a substance (protein, low-molecular-weight compound, nucleic acid, or the like), which cannot normally pass through the blood brain barrier and thus cannot exert a physiological or pharmacological action in the brain in the case of intravenous administration. Such a substance can be bound to the conjugate of the Fc region and the hTfR affinity peptide by the above-described method, for example, a method using PEG. It is predicted that, in a case where the complex is administered to a living body by intravenous injection or the like, the complex can pass through the blood brain barrier and reach the cerebral cortex of the cerebrum, the neuron-like cells of the hippocampus, the cerebral cortex of the cerebellum, and the like. Therefore, by using the conjugate, it is possible to exhibit the physiological activity of these substances in the tissue or the cell of the central nervous system. In addition, since the stability in blood is higher than that of the complex having no hIgG Fc region in blood, in a case of being administered to a living body, the pharmaceutical effect can be exhibited for a longer period of time. A protein in which the hTfR affinity peptide is bound to the Fc region of human IgG can be obtained as a recombinant protein, similarly to the hTfR affinity peptide-protein (A) fusion protein.

The hTfR affinity peptide and a different protein (A) can also be bound to an hIgG Fc region. By binding the hIgG Fc region, stability of the conjugate in a living body, for example, blood retention can be increased. Examples of such a protein to which the Fc region is bound include a protein in which the hIgG Fc region is directly or via a peptide linker bound to the C-terminus of the different protein (A), and the hTfR affinity peptide is directly or via a peptide linker bound to the C-terminus of the hIgG Fc region. In addition, such a protein includes a protein in which the hIgG Fc region is directly or via a peptide linker bound to the C-terminus of the hTfR affinity peptide, and further, the different protein (A) is directly or via a linker sequence bound to the C-terminus of the hIgG Fc region. In addition, such a protein includes a protein in which the different protein (A) is directly or via a peptide linker bound to the C-terminus of the hTfR affinity peptide, and further, the hIgG Fc region is directly or via a linker sequence bound to the C-terminus of the different protein (A). In addition, such a protein includes a protein in which the hTfR affinity peptide is directly or via a peptide linker bound to the C-terminus of the different protein (A), and further, the hIgG Fc region is directly or via a linker sequence bound to the C-terminus of the hTfR affinity peptide. In addition, such a protein includes a protein in which the hTfR affinity peptide is directly or via a peptide linker bound to the C-terminus of the hIgG Fc region, and further, the different protein (A) is directly or via a linker sequence bound to the C-terminus of the hTfR affinity peptide. Such a protein includes a protein in which the different protein (A) is directly or via a peptide linker bound to the C-terminus of the hIgG Fc region, and the hTfR affinity peptide is directly or via a linker sequence bound to the C-terminus of the different protein (A). The protein in which the hTfR affinity peptide, the different protein (A), and the hIgG Fc region are bound to each other can be obtained as a recombinant protein, similarly to the hTfR affinity peptide-protein (A) fusion protein. The recombinant protein thus obtained is also included in the hTfR affinity peptide-protein (A) fusion protein. As the peptide linker which can be used here, a peptide linker disposed between the above-described hTfR affinity peptide and different protein (A) can be used.

The type of IgG of the hIgG Fc region to be bound is not particularly limited, and may be any of IgG1 to IgG5. In addition, the introduced hIgG Fc region may be the entire Fc region or a part of the Fc region. Examples of a preferred embodiment of such an hIgG Fc region include an hIgG Fc region having the amino acid sequence set forth in SEQ ID NO: 26, which is the entire region of the Fc of human IgG1.

The hTfR affinity peptide can also be bound to human serum albumin (HSA). By binding HSA to the hTfR affinity peptide, the stability of the hTfR affinity peptide in vivo, for example, the blood retention can be enhanced. Examples of such hTfR affinity peptide to which HSA is bound include a peptide in which HSA is directly or via a peptide linker bound to the C-terminus of the hTfR affinity peptide, and a peptide in which HSA is directly or via a peptide linker bound to the N-terminus of the hTfR affinity peptide. The conjugate of HSA and the hTfR affinity peptide can be used to form a complex in which the conjugate is further bound to a substance (protein, low-molecular-weight compound, nucleic acid, or the like), which cannot normally pass through the blood brain barrier and thus cannot exert a physiological or pharmacological action in the brain by intravenous administration. Such a substance can be bound to the conjugate of HSA and the hTfR affinity peptide by the above-described method, for example, a method using PEG. It is predicted that, in a case where the complex is administered to a living body by intravenous injection or the like, the complex can pass through the blood brain barrier and reach the cerebral cortex of the cerebrum, the neuron-like cells of the hippocampus, the cerebral cortex of the cerebellum, and the like. Therefore, by using the conjugate, it is possible to exhibit the physiological activity of these substances in the tissue or the cell of the central nervous system. In addition, since the stability in blood of the complex is higher than the stability in blood of the complex having no HSA, in a case of being administered to a living body, the drug efficacy can be exhibited for a longer period of time. A protein in which the hTfR affinity peptide is bound to HSA can be obtained as a recombinant protein, similarly to the hTfR affinity peptide-protein (A) fusion protein.

HSA can also be further bound to the hTfR affinity peptide-protein (A) fusion protein. Examples of such a protein to which HSA is bound include a protein in which HSA is directly or via a peptide linker bound to the C-terminus of the different protein (A), and further the hTfR affinity peptide is directly or via a peptide linker bound to the C-terminus of the HSA. In addition, such a protein includes a protein in which HSA is directly or via a peptide linker bound to the C-terminus of the hTfR affinity peptide, and further, the different protein (A) is directly or via a linker sequence bound to the C-terminus of the HSA. In addition, such a protein includes a protein in which the different protein (A) is directly or via a peptide linker bound to the C-terminus of the hTfR affinity peptide, and further, HSA is directly or via a linker sequence bound to the C-terminus of the different protein (A). In addition, such a protein includes a protein in which the hTfR affinity peptide is directly or via a peptide linker bound to the C-terminus of the different protein (A) and further, HSA is directly or via a linker sequence bound to the C-terminus of the hTfR affinity peptide. In addition, such a protein includes a protein in which the hTfR affinity peptide is directly or via a peptide linker bound to the C-terminus of HSA and further, the different protein (A) is directly or via a linker sequence bound to the C-terminus of the hTfR affinity peptide. In addition, such a protein includes a protein in which the different protein (A) is directly or via a peptide linker bound to the C-terminus of the HSA, and further the hTfR affinity peptide is directly or via a linker sequence bound to the C-terminus of the different protein (A). A protein obtained by binding the hTfR immunoglobulin single variable domain peptide to the different protein (A) and HSA can be obtained as a recombinant protein, similarly to the hTfR immunoglobulin single variable domain peptide-protein (A) fusion protein. The recombinant protein thus obtained is also included in the hTfR affinity peptide-protein (A) fusion protein. As the peptide linker which can be used here, a peptide linker disposed between the above-described hTfR affinity peptide and different protein (A) can be used.

The above-described HSA is preferably a wild type HSA having the amino acid sequence shown in SEQ ID NO: 27, but is not limited thereto, and as long as the stability of the substance bound to the HSA in blood can be improved, a mutant HSA in which a substitution, a deletion, or an addition is added to the amino acid sequence of the wild type HSA may be used.

A substance having an affinity for albumin can also be used instead of HSA. Since a substance having an affinity for albumin binds to albumin in blood, the half-life in blood of the fusion protein of the anti-hTfR heavy chain antibody and the different protein (A) or the like administered to a living body can be increased in the same manner as in a case of using HSA. As the substance having an affinity for albumin, for example, a peptide obtained by modifying an albumin binding domain of a protein derived from Streptococcus strain G418 having an amino acid sequence represented by SEQ ID NO: 28 to exhibit alkali resistance (Alm T. Biotechnol J. 5. 605-17 (2010)) can be used.

The binding affinity of the albumin with the hTfR affinity peptide-protein (A) fusion protein into which the albumin affinity peptide has been introduced, which is measured in accordance with the bio-layer interferometry method described in Example 7, is preferably 1 × 10⁻⁷ M or less, more preferably 5 × 10⁻⁷ M or less, still more preferably 1 × 10⁻⁸ M or less, and even still more preferably 1 × 10⁻⁹ M or less.

A plurality of hTfR affinity peptides can also be bound directly or via a peptide linker. In this case, the plurality of hTfR affinity peptides to be bound to each other may all have the same amino acid sequence, or may have different amino acid sequences. The number of the hTfR affinity peptides to be bound is not particularly limited, but is, for example, 2 to 10, 2 to 5, 2, 3, or the like. By binding a plurality of hTfR affinity peptides, it is possible to impart properties such as improvement of the affinity for anti-hTfR. In the present specification, a peptide in which a plurality of hTfR affinity peptides are linked in this manner is referred to as a tandem type hTfR affinity peptide. The tandem type hTfR affinity peptide is included in the hTfR affinity peptide. The same can be said for the immunoglobulin single variable domain peptide and the anti-hTfR heavy chain antibody variable region peptide.

The peptide linker used for linking the above-described hTfR affinity peptide with the hIgG Fc region or HSA is preferably composed of 1 to 60 or 1 to 50 amino acids. Here, the number of amino acids is appropriately adjusted to 1 to 17, 1 to 10, 10 to 40, 20 to 34, 23 to 31, 25 to 29, 27, or the like. Such a peptide linker is not limited as long as the hTfR affinity peptide linked thereto has an affinity for hTfR and the hIgG Fc region or HSA linked by the peptide linker can exhibit a desired function under physiological conditions, and is preferably composed of glycine and serine. The same applies to the peptide linker used for binding the hTfR affinity peptide, the different protein (A), and the hIgG Fc region. The same applies to the peptide linker used for binding the hTfR affinity peptide, the different protein (A), and HSA. The same applies to the peptide linker for binding a plurality of hTfR affinity peptides.

Examples of the preferred peptide linker include a peptide linker consisting of one amino acid (for example, glycine or serine), the amino acid sequence Gly-Ser, the amino acid sequence Ser-Ser, the amino acid sequence Gly-Gly-Ser, the amino acid sequence set forth in SEQ ID NO: 19, the amino acid sequence set forth in SEQ ID NO: 20, the amino acid sequence set forth in SEQ ID NO: 21, the amino acid sequence set forth in SEQ ID NO: 22, the amino acid sequence set forth in SEQ ID NO: 23, the amino acid sequence set forth in SEQ ID NO: 24, and the amino acid sequence set forth in SEQ ID NO: 25. In addition, examples of the preferred peptide linker include a peptide linker consisting of one amino acid (for example, glycine or serine), a peptide linker including a sequence in which 2 to 10 or 2 to 5 amino acid sequences selected from the group consisting of the amino acid sequence Gly-Ser, the amino acid sequence Ser-Ser, the amino acid sequence Gly-Gly-Ser, the amino acid sequence of SEQ ID NO: 19, the amino acid sequence of SEQ ID NO: 20, and the amino acid sequence of SEQ ID NO: 21 are arranged in series. The peptide linker has a sequence consisting of 2 to 50 amino acids, a sequence consisting of 2 to 17 amino acids, 2 to 10 amino acids, 10 to 40 amino acids, 20 to 34 amino acids, 23 to 31 amino acids, 25 to 29 amino acids, or 27 amino acids. For example, a compound including the amino acid sequence Gly-Ser can be suitably used as the peptide linker. In addition, a peptide linker including a total of 25 amino acids in which the amino acid sequence set forth in SEQ ID NO: 19 is repeated 5 times can also be suitably used as the peptide linker.

The hTfR affinity peptide and the hTfR affinity peptide-protein (A) fusion protein can also be stabilized in blood by a method other than the above. For example, these proteins can be modified with PEG to enhance stability in blood. Such a method is generally carried out in the field of protein medicine, and PEGylated erythropoietin, interferon, and the like have been put into practical use as pharmaceuticals. In addition, the hTfR affinity peptide can also be stabilized by introducing a mutation into the hTfR affinity peptide.

The different protein (A) to be bound to the hTfR affinity peptide are not particularly limited, but is a protein that can exhibit physiological activity in vivo, and, in particular, is a protein that are not expected to function in the brain by intravenous administration since it cannot pass through the blood brain barrier as they are, although it needs to reach the brain and exhibit the function thereof. Examples of such a protein includes lysosomal enzymes such as iduronate-2-sulfatase (IDS), glucocerebrosidase (GBA), β-galactosidase, GM2-activator protein, β-hexosaminidase A, β-hexosaminidase B, N-acetylglucosamine-1-phosphotransferase, α-mannosidase (LAMAN), β-mannosidase, galactosylceramidase (GALC), saposin C, arylsulfatase A (ARSA), α-L-fucosidase (FUCA1), aspartylglucosaminidase, α-N-acetylgalactosaminidase, acid sphingomyelinase (ASM), α-galactosidase A, β-glucuronidase (GUSB), heparan N-sulfatase (SGSH), α-N-acetylglucosaminidase (NAGLU), acetyl-CoA:α-glucosaminide N-acetyltransferase, N-acetylglucosamine-6-sulfatase, acid ceramidase (AC), amylo-1,6-glucosidase, sialidase, aspartylglucosaminidase, palmitoyl-protein thioesterase-1 (PPT-1), tripeptidyl peptidase-1 (TPP-1), hyaluronidase-1, acid α-glucosidase (GAA), CLN1, and CLN2. The same can be applied to the variable region peptide of the anti-hTfR heavy chain antibody.

The hTfR affinity peptide bound to α-L-iduronidase (IDUA) can be used as a therapeutic agent for Hurler syndrome or Hurler-Scheie syndrome, the hTfR affinity peptide bound to iduronate-2-sulfatase (IDS) can be used as a therapeutic agent for Hunter syndrome, the hTfR affinity peptide bound to glucocerebrosidase (GBA) can be used as a therapeutic agent for Gaucher disease, the hTfR affinity peptide bound to β-galactosidase can be used as a therapeutic agent for GM1-gangliosidosis type 1 to 3, the hTfR affinity peptide bound to GM2-activator protein can be used as a therapeutic agent for GM2-gangliosidosis AB variant, the hTfR affinity peptide bound to β-hexosaminidase A can be used as a therapeutic agent for Sandhoff disease and Tay-Sachs disease, the hTfR affinity peptide bound to β-hexosaminidase B can be used as a therapeutic agent for Sandhoff disease, the hTfR affinity peptide bound to N-acetylglucosamine-1-phosphotransferase can be used as a therapeutic agent for I-cell disease, the hTfR affinity peptide bound to α-mannosidase (LAMAN) can be used as a therapeutic agent for α-mannosidosis, the hTfR affinity peptide bound to β-mannosidase can be used as a therapeutic agent for β-mannosidosis, the hTfR affinity peptide bound to galactosylceramidase (GALC) can be used as a therapeutic agent for Krabbe disease, the hTfR affinity peptide bound to saposin C can be used as a therapeutic agent for Gaucher-like accumulation disease, the hTfR affinity peptide bound to arylsulfatase A (ARSA) can be used as a therapeutic agent for metachromatic leukodystrophy, the hTfR affinity peptide bound to α-L-fucosidase (FUCA1) can be used as a therapeutic agent for fucosidosis, the hTfR affinity peptide bound to aspartylglucosaminidase can be used as a therapeutic agent for aspartylglucosaminuria, the hTfR affinity peptide bound to α-N-acetylgalactosaminidase can be used as a therapeutic agent for Chindler disease and Kawasaki disease, the hTfR affinity peptide bound to acid sphingomyelinase (ASM) can be used as a therapeutic agent for Niemann-Pick disease, the hTfR affinity peptide bound to α-galactosidase A can be used as a therapeutic agent for Fabry disease, the hTfR affinity peptide bound to β-glucuronidase (GUSB) can be used as a therapeutic agent for Sly syndrome, the hTfR affinity peptide bound to heparan N-sulfatase (SGSH) can be used as a therapeutic agent for Sanfilippo syndrome A, the hTfR affinity peptide bound to α-N-acetylglucosaminidase (NAGLU) can be used as a therapeutic agent for Sanfilippo syndrome B, the hTfR affinity peptide bound to acetyl-CoAα-glucosaminide N-acetyltransferase and N-acetylglucosamine-6-sulfatase can be used as a therapeutic agent for Sanfilippo syndrome, the hTfR affinity peptide bound to acid ceramidase (AC) can be used as a therapeutic agent for Farber disease, the hTfR affinity peptide bound to amylo-1,6-glucosidase can be used as a therapeutic agent for Cori disease (Forbes-Cori disease), the hTfR affinity peptide bound to sialidase can be used as a therapeutic agent for central nervous system disorder in sialidase deficiency, the hTfR affinity peptide bound to palmitoyl-protein thioesterase-1 (PPT-1) can be used as a therapeutic agent for neuronal ceroid lipofuscinosis or Santavuori-Haltia disease, the hTfR affinity peptide bound to tripeptidyl peptidase-1 (TPP-1) can be used as a therapeutic agent for neuronal ceroid lipofuscinosis or Jansky-Bielschowsky disease, the hTfR affinity peptide bound to hyaluronidase-1 can be used as a therapeutic agent for hyaluronidase deficiency, the hTfR affinity peptide bound to acid α-glucosidase (GAA) can be used as a therapeutic agent for Pompe disease, and the hTfR affinity peptide bound to CLN1 and 2 can be used as a therapeutic agent for each of Batten disease. The hTfR affinity peptide bound to the lysosomal enzyme can be used as a therapeutic agent for a central nervous disorder in lysosomal disease in particular. For example, an hTfR affinity peptide bound to α-L-iduronidase (IDUA) can be used as a therapeutic agent for central nervous system disorder in Hurler syndrome or Hurler-Scheie syndrome. The medical application is not limited to these diseases.

Examples of the protein which can exhibit a drug efficacy by binding to the hTfR affinity peptide include a growth factor, an antibody drug, a cytokine, a nerve growth factor (NGF), a brain-derived nerve growth factor (BDNF), a ciliary neurotrophic factor (CNTF), a glial cell line-derived neurotrophic factor (GDNF), a neurotrophin 3, a neurotrophin 4/5, a neurotrophin 6, a neuregulin 1, an erythropoietin, a darbepoietin, an activin, a basic fibroblast growth factor (bFGF), a fibroblast growth factor 2 (FGF2), an epithelial cell growth factor (EGF), a vascular endothelial growth factor (VEGF), an interferon α, an interferon β, an interferon γ, an interleukin 6, a granulocyte-macrophage colony-stimulating factor (GM-CSF), a granulocyte colony-stimulating factor (G-CSF), a macrophage colony-stimulating factor (M-CSF), a tumor necrosis factor α receptor (TNF-αR), a PD-1 ligand, PD-L1, PD-L2, an enzyme having an activity of degrading β-amyloid, an anti-β-amyloid antibody, an anti-BACE antibody, an anti-EGFR antibody, an anti-PD-1 antibody, an anti-PD-L1 antibody, an anti-PD-L2 antibody, an anti-HER2 antibody, an anti-TNF-α antibody, and an anti-CTLA-4 antibody.

The hTfR affinity peptide binding to a nerve growth factor (NGF) can be used as a therapeutic agent for a neurologic disorder of Alzheimer's disease, the hTfR affinity peptide binding to a brain-derived growth factor (BDNF) can be used as a therapeutic agent for a neurologic disorder such as Huntington's disease and Alzheimer's disease, the hTfR affinity peptide binding to CNTF can be used as a therapeutic agent for amyotrophic lateral sclerosis, the hTfR affinity peptide binding to GDNF, neurotrophin 3, or neurotrophin 4/5 can be used as a therapeutic agent for cerebral ischemia, the hTfR affinity peptide binding to GDNF can be used as a therapeutic agent for Parkinson's disease, the hTfR affinity peptide binding to neuregulin 1 can be used as a therapeutic agent for schizophrenia, the hTfR affinity peptide binding to erythropoietin or darbepoietin can be used as a therapeutic agent for cerebral ischemia, the hTfR affinity peptide binding to bFGF or FGF2 can be used as a therapeutic agent for traumatic central nervous system disorders, for recovery after brain surgery or spinal surgery, the hTfR affinity peptide binding to an enzyme having an activity of degrading β-amyloid, an anti-β-amyloid antibody, or an anti-BACE antibody can be used as a therapeutic agent for Alzheimer's disease, the hTfR affinity peptide binding to an anti-EGFR antibody, an anti-PD-1 antibody, an anti-PD-L1 antibody, an anti-PD-L2 antibody, an anti-HER2 antibody, or an anti-CTLA-4 antibody can be used as a therapeutic agent for a tumor of the central nervous system including a brain tumor, and the hTfR affinity peptide binding to TNFαR can be used as a therapeutic agent for cerebral ischemia and encephalitic diseases.

A therapeutic agent for a neurodegenerative disease such as Alzheimer's disease, Parkinson's disease, and Huntington's disease, a mental disorder such as schizophrenia and depression, a tumor of the central nervous system including multiple sclerosis, amyotrophic lateral sclerosis, and brain tumor, a lysosomal disease accompanied by brain disorder, a glycogen storage disease, a muscular dystrophy, cerebral ischemia, an encephalitic disease, a prion disease, a traumatic central nervous system disorder, or the like can be a different protein (A) to be fused with the hTfR affinity peptide in general. In addition, a therapeutic agent for a viral and bacterial central nervous system disease can also be a different protein (A) to be fused with the hTfR affinity peptide in general. Furthermore, the drug that can also be used for recovery after a neurosurgery or a spinal surgery can be a different protein (A) to be fused with the hTfR affinity peptide in general.

In the present invention, the therapeutic agent also includes a therapeutic agent used for preventing the onset of a disease.

The proteins listed above as the different protein (A) to be fused to the hTfR affinity peptide are usually wild-type proteins. However, a variant in which one or more amino acids constituting these wild-type proteins are substituted with other amino acids, deleted, or the like is also included in these proteins as long as the variant has the original physiological activity of the protein. Here, "the protein has the original physiological activity" means that the protein has 20% or more physiological activity with respect to the physiological activity of the wild-type protein. The physiological activity with respect to the physiological activity of the wild-type protein is more preferably 40% or more, still more preferably 50% or more, still more preferably 80% or more, and still more preferably 90% or more.

An amino acid sequence of the variant protein has preferably 80% or more, more preferably 85% or more, still more preferably 90% or more, and even still more preferably 95% and for example, 98% or more identity with the amino acid sequence of the corresponding wild-type protein (A).

In a case where the amino acid sequence of the wild-type different protein (A) is substituted with another amino acid to obtain a variant, the number of the amino acids to be substituted is preferably 1 to 10, more preferably 1 to 5, and still more preferably 1 to 3. In a case where the amino acid is deleted, the number of the amino acids to be deleted is preferably 1 to 10, more preferably 1 to 5, and still more preferably 1 to 3. In addition, a desired variant can also be obtained by combining the substitution and the deletion. Furthermore, a protein in which one or more amino acids are added to the wild-type protein (A) or a variant thereof in the amino acid sequence, the N-terminus, or the C-terminus is also included in these proteins as long as the protein has the function as a protein completely or partially. The number of amino acids to be added at this time is preferably 1 to 10, more preferably 1 to 5, and still more preferably 1 to 3. A desired variant can also be obtained by combining addition, substitution, and deletion of these amino acids.

In a case where a mutation is applied to the different protein (A) and an amino acid is added to the C-terminus or the N-terminus, the added amino acid is considered to be a part of the different protein (A) in a case where the added amino acid is positioned between the different protein (A) and the hTfR affinity peptide when the different protein (A) is fused with the hTfR affinity peptide.

Examples of a suitable fusion protein in a case where the different protein (A) is human IDS include the following (1) to (4):
(1) a fusion protein in which the N-terminus of hIDS is bound to the C-terminus of the hTfR affinity peptide consisting of the amino acid sequence set forth in SEQ ID NO: 4, SEQ ID NO: 5, or SEQ ID NO: 6, via the peptide linker having the amino acid sequence set forth in SEQ ID NO: 25,
(2) a fusion protein in which the N-terminus of hIDS is bound to the C-terminus of the hTfR affinity peptide 2 consisting of the amino acid sequence set forth in SEQ ID NO: 5 via the peptide linker having the amino acid sequence set forth in SEQ ID NO: 25,
(3) a fusion protein in which the N-terminus of the hTfR affinity peptide 2 consisting of the amino acid sequence set forth in SEQ ID NO: 5 is bound to the C-terminus of the hTfR affinity peptide 2 consisting of the amino acid sequence set forth in SEQ ID NO: 5 via the peptide linker having the amino acid sequence set forth in SEQ ID NO: 25, and further to the C-terminus thereof the N-terminus of the hIDS is bound via the peptide linker having the amino acid sequence GS.

Examples of a suitable fusion protein in a case where the different protein (A) is human SGSH include the following. That is,
(1) a fusion protein in which the N-terminus of hSGSH is bound to the C-terminus of the hTfR affinity peptide consisting of an amino acid sequence set forth in SEQ ID NO: 4, SEQ ID NO: 5, or SEQ ID NO: 6, via the peptide linker having the amino acid sequence set forth in SEQ ID NO: 25,
(2) a fusion protein in which the N-terminus of hSGSH is bound to the C-terminus of the hTfR affinity peptide 2 consisting of the amino acid sequence set forth in SEQ ID NO: 7 via the peptide linker having the amino acid sequence set forth in SEQ ID NO: 25.

Trastuzumab is an antibody drug that exhibits an antitumor effect by specifically binding to HER2 encoded by a human cancer gene HER2/neu (c-erbB-2). Trastuzumab consists of a heavy chain and a light chain, and the amino acid sequences thereof are shown in SEQ ID NOs: 29 and 30, respectively.

Specific examples of the fusion protein of the hTfR affinity peptide and the different protein (A) in the present invention include a fusion protein of the hTfR affinity peptide and trastuzumab, in which the hTfR affinity peptide 1 is bound to the C-terminus of the heavy chain of trastuzumab via a linker. The fusion protein of hTfR affinity peptide 1 and trastuzumab can be produced, for example, by culturing a host cell into which an expression vector of a protein (hTfR affinity peptide 1-trastuzumab heavy chain fusion protein) having the amino acid sequence of SEQ ID NO: 31 in which the hTfR affinity peptide 1 having the amino acid sequence of SEQ ID NO: 4 is bound to the C-terminus of the heavy chain of trastuzumab via the peptide linker having the amino acid sequence of SEQ ID NO: 24 and an expression vector of a protein of a light chain of trastuzumab have been introduced. By using an expression vector of the hTfR affinity peptide 2-trastuzumab heavy chain fusion protein having the amino acid sequence set forth in SEQ ID NO: 32 or an expression vector of the hTfR affinity peptide 3-trastuzumab heavy chain fusion protein having the amino acid sequence set forth in SEQ ID NO: 33, instead of the expression vector of the hTfR affinity peptide 1-trastuzumab heavy chain fusion protein, it is also possible to produce a fusion protein of another embodyment of the hTfR affinity peptide and trastuzumab.

Trastuzumab is mainly used as a therapeutic agent for breast cancer, but in a case of intravenous injection, it cannot pass through the BBB, and thus cannot exhibit a drug efficacy on cancer of the central nervous system. However, by binding trastuzumab to the anti-hTfR heavy chain antibody variable region peptide, the trastuzumab can pass through the BBB, and thus can act on a cancer expressing HER2 in the central nervous system. Since breast cancer may metastasize to the central nervous system, the fusion protein of the TfR affinity peptide and trastuzumab can be expected to be effective for the cancer of the central nervous system metastasized from such breast cancer.

The antibody drug other than trastuzumab can also be produced as a fusion protein with the anti-hTfR heavy chain antibody variable region peptide in the same manner as trastuzumab. In this case, the anti-hTfR heavy chain antibody variable region peptide may be bound to the C-terminus of the heavy chain of the antibody, which is the active ingredient of the antibody drug, may be bound to the N-terminus of the heavy chain of the antibody, may be bound to the C-terminus of the light chain of the antibody, or may be bound to the N-terminus of the light chain of the antibody.

The drug to be form a complex with the hTfR affinity peptide may be a low-molecular-weight compound. Here, the low-molecular-weight compound is preferably a compound which should pass through the BBB and act on the central nervous system, but is not limited thereto, and may be a contrast agent, a fluorescent colorant, a phospholipid which can be a constituent of liposomes, a functional lipid which can be a constituent of lipid nanoparticles, or the like. The molecular weight of the low-molecular-weight compound is, for example, 1 to 50 kD, 0.5 to 20 kD, or 1 to 5 kD. A peptide consisting of 5 to 20 amino acids and a polysaccharide having a structure in which 5 to 20 monosaccharides are dehydratively condensed are also included in the low-molecular-weight compound.

The hTfR affinity peptide and the low-molecular-weight compound are bound to each other by a covalent bond directly or via a linker. A binding mode of the hTfR affinity peptide and the low-molecular-weight compound is not particularly limited as long as the binding affinity of the hTfR affinity peptide to hTfR is maintained and the drug exhibits a drug efficacy in the central nervous system in a case where the hTfR affinity peptide-drug complex is administered to a living body. As the linker, for example, a linker containing valine-citrulline or valine-alanine, which is cleaved by cathepsin B, or a linker containing cysteine-cysteine is suitably used. In a case of being administered to a living body, such a linker can be cleaved by a metabolic mechanism, for example, an enzyme, in the living body so that the low-molecular-weight compound can be separated from the hTfR affinity peptide and can exhibit a drug efficacy alone. In the present specification, such a linker that can be cleaved in a living body is referred to as a biodegradable linker.

In a case where the hTfR affinity peptide and the low-molecular-weight compound are bound to each other by the biodegradable linker, it is possible to dispose a spacer in addition to the linker between these. By disposing the spacer, accessibility to the complex by the enzyme that cleaves the linker is improved. Such a spacer is not particularly limited, but a p-aminocarbamate group/carbamate group is suitably used.

The drug in the hTfR affinity peptide-drug complex may be a nucleic acid. Here, the nucleic acid may be any of single-stranded DNA, double-stranded DNA, single-stranded RNA, or double-stranded RNA. In order to prevent the nucleic acid from being degraded by a nuclease in a living body, the nucleotide constituting these nucleic acids can be modified to be a nuclease-resistant nucleic acid. The chain length of the nucleic acid is not particularly limited, but is, for example, 10 bp to 1,000 bp, or 10 bp to 100 bp. In the complex, the hTfR affinity peptide and the nucleic acid are bound to each other by a covalent bond directly or via a linker. A binding mode of the immunoglobulin single variable domain and the low-molecular-weight compound is not particularly limited as long as the binding affinity of the hTfR affinity peptide to hTfR is maintained and the drug exhibits a drug efficacy in the central nervous system in a case where the hTfR affinity peptide-nucleic acid complex is administered to a living body. As the linker, a biodegradable linker is preferably used.

In a case where the hTfR affinity peptide and the nucleic acid are bound to each other by a biodegradable linker, it is possible to dispose a spacer in addition to the linker between these. By disposing the spacer, accessibility to the nucleic acid complex to be cleaved the linker is improved. Such a spacer is not particularly limited, but a p-aminocarbamate group/carbamate group is suitably used.

The nucleic acid to be bound to the hTfR affinity peptide is, for example, a nucleic acid used for the treatment of a central nervous system disorder. Such a nucleic acid is an antisense nucleic acid to mRNA, the expression of which is abnormally enhanced in the central nervous system. Such antisense nucleic acid exhibits an effect of binding to an abnormally expressed mRNA to inhibit translation of the mRNA into a protein.

The hTfR affinity peptide-drug complex (including the hTfR affinity peptide-different protein (A) fusion protein) can be supplied to a medical institution in a form of a freeze-dried product, an aqueous liquid agent, or the like as a drug (pharmaceutical composition) containing the hTfR affinity peptide-drug complex as an active ingredient. In a case of the aqueous liquid agent, the hTfR affinity peptide-drug complex can be supplied as a formulation in which the hTfR affinity peptide-drug complex is dissolved in advance in a solution containing a stabilizer, a buffer, an isotonizing agent, or the like and is enclosed in a vial or a syringe. The agent enclosed in the syringe is generally referred to as a pre-filled syringe agent. By using the pre-filled syringe agent, it is possible to simplify the administration of the drug by the patient himself/herself. The administration of the drug is performed by a non-oral method, for example, intravenous injection. The administered drug can pass through the blood brain barrier and exhibit a drug efficacy in the central nervous system, and can exhibit a drug efficacy in the cerebral cortex, the cerebellum, the hippocampus, the midbrain, the pons, the medulla oblongata, the caudate nucleus, the putamen, the globus pallidus, the thalamus, and the hypothalamus, and can exhibit a drug efficacy in the cerebral cortex, the cerebellum, the hippocampus, and the midbrain in particular.

The hTfR affinity peptide can be used to modify the surface of a vesicle that can encapsulate a drug. Examples of the vesicle include liposomes and lipid nanoparticles (LNP). The liposome is a spherical vesicle having a lipid bilayer, and a liposome containing a phospholipid, particularly phosphatidylcholine, as a constituent is generally used. The liposome may further contain other lipids such as egg yolk phosphatidylethanolamine as a constituent component. The lipid nanoparticles refer to particles having a diameter of 10 nm to 1000 nm, typically less than about 200 nm, and having lipids as main components, and can encapsulate a hydrophobic (lipophilic) molecule, and have lipids compatible with a living body, such as triglyceride, diglyceride, monoglyceride, fatty acid, and steroid, as main constituent components. The drug can be enclosed in these vesicles. A lipid nanoparticle in which a surface is modified with the hTfR affinity peptide can be produced by producing a constituent of a lipid nanoparticle to which an hTfR affinity peptide is bound, and adding it in a production process of the lipid nanoparticle. The same applies to the liposome.

In a case where the vesicle enclosing the drug is administered to a living body, a membrane constituting the vesicle fuses with a cell membrane, and the drug is released into a cell. That is, these vesicles can be used as a carrier of a drug. In a case where the hTfR affinity peptide is disposed on the surface of such a vesicle, the vesicle binds to the hTfR on the brain microvascular endothelial cell constituting the blood brain barrier via the peptide, and the drug enclosed in the vesicle can be released into the brain parenchyma such as the cerebellar parenchyma by the vesicle being fused with the cell membrane of the brain microvascular endothelial cell or the vesicle being taken into the cell by endocytosis or the like.

In the present specification, the term "vesicle" refers to not only the above-described liposome and lipid nanoparticles but also any nano- or micro-particles that can be used as a drug delivery system (DDS), such as polymer nanoparticles, micelles, emulsions, nanoemulsions, microspheres, nanospheres, microcapsules, nanocapsules, dendrimers, nanogels, metal nanoparticles, and the like.

### Examples

Hereinafter, the present invention will be described in more detail with reference to Examples, but the present invention is not limited to Examples.

### [Reference Example 1] Production of hTfR affinity peptide-trastuzumab (reference product) expression vector

A known fusion protein of a hTfR affinity peptide and trastuzumab was produced by the method shown in Reference Examples 1 and 2 for use as a reference product. The fusion protein has the same amino acid sequence as that disclosed as hTfR affinity peptide-trastuzumab 5 in WO2023/090409A.

pCI-neo Mammalian Expression Vector (Promega Corporation) was digested with MluI and NotI. A DNA fragment of a gene that has the nucleotide sequence set forth in SEQ ID NO: 44 and encodes a protein having the amino acid sequence set forth in SEQ ID NO: 43 in which an hTfR affinity peptide 5 represented in SEQ ID NO: 42 was bound to the C-terminus of a trastuzumab heavy chain having the amino acid sequence set forth in SEQ ID NO: 29 via a peptide linker having the amino acid sequence set forth in SEQ ID NO: 24, the DNA fragment having a MluI site on the 5' side and a NotI site on the 3' side of the gene, was artificially synthesized. The expression vector of a protein in which the hTfR affinity peptide 5 and the trastuzumab heavy chain were bound to each other was produced by digesting the DNA fragment with MluI and NotI and incorporating the digested DNA fragment between the MluI site and the NotI site of the pCI-neo Mammalian Expression Vector. This expression vector was named pCI-neo-Trastuzumab(HC)-VHH(5).

In addition, a DNA fragment of a gene that has the nucleotide sequence set forth in SEQ ID NO: 35 and encodes the light chain of trastuzumab having the amino acid sequence set forth in SEQ ID NO: 30, the DNA fragment having a MluI site on the 5' side and a NotI site on the 3' side of the gene, was artificially synthesized. The expression vector of the light chain of trastuzumab was constructed by digesting the DNA fragment with MluI and NotI, and ligating the digested DNA fragment with the pCI-neo Mammalian Expression Vector (Promega Corporation) digested with MluI and NotI, which was prepared in advance. The expression vector was named pCI-neo-Trastuzumab(LC).

### [Reference Example 2] Production of hTfR affinity peptide-trastuzumab (reference product)

pCI-neo-Trastuzumab(HC)-VHH(5) and pCI-neo-Trastuzumab(LC), which were produced in Reference Example 1, were simultaneously transfected to CHO-S cells using ExpiCHO Expression System (Thermo Fisher Scientific, Inc.) according to the attached protocol, and the protein in which the hTfR affinity peptide 5 and the heavy chain of trastuzumab were bound to each other and the light chain of trastuzumab were co-expressed in CHO-S cells. This cell expresses the protein in which the hTfR affinity peptide 5 and trastuzumab are fused to each other, and secretes the protein into a culture medium. Hereinafter, this fusion protein is referred to as hTfR affinity peptide-trastuzumab 5. This cell was cultured in the presence of neomycin according to the attached protocol. Specifically, CHO-S cells were suspended in 15 mL of a culture medium such that the concentration was 6 × 10⁶ cells/mL, the suspension was transferred to a 150 mL conical flask, and the cells were cultured at 37°C in a wet environment consisting of 5% CO₂ and 95% air at a stirring speed of about 120 rpm for 1 day. The feed liquid and the enhancer liquid attached to the ExpiCHO Expression System were added thereto, and the cells were cultured at 32°C in a wet environment consisting of 5% CO₂ and 95% air at a stirring speed of about 120 rpm for 9 days to express the hTfR affinity peptide-trastuzumab 5. After the culture, the culture medium was centrifuged (3,000 xg, 5 minutes) to obtain a supernatant, the supernatant was filtered through a 0.22 µm filter (MilliporeSigma), the filtrate was collected as a culture supernatant.

An open column filled with 1 mL of MabSelect SuRe LX (Cytiva) was equilibrated by passing through 5 column volumes of 25 mM HEPES buffer solution (pH 7.5) containing 50 mM NaCl. Next, the culture supernatant was applied onto the column, and the hTfR affinity peptide-trastuzumab 5 was bound to the resin. The column was washed by passing through 5 column volumes of 25 mM HEPES buffer solution (pH 7.5) containing 50 mM NaCl, and then hTfR affinity peptide-trastuzumab 5 was eluted by passing 150 mM Glycine (pH 3.0) containing 100 mM NaCl. 1 M Tris-HCl (pH 8.0) equivalent to 5% by volume was added to the eluate containing the hTfR affinity peptide-trastuzumab 5 to adjust the pH to neutral. The solvent of the eluate was buffer-exchanged with PBS(-) using a PD-10 column (Cyteva Inc.). The obtained solution was used as a purified product of the hTfR affinity peptide-trastuzumab 5, and this was used as hTfR affinity peptide-trastuzumab (reference product).

### [Example 1] Construction of hTfR expression vector

Using human spleen Quick Clone cDNA (Clontech) as a template, a gene fragment encoding a human transferrin receptor (hTfR) was amplified by PCR using a primer hTfR5' (SEQ ID NO: 36) and a primer hTfR3' (SEQ ID NO: 37). The amplified gene fragment encoding hTfR was digested with MluI and NotI, and the digested fragment was inserted between MluI and NotI of a pCI-neo vector (Promega Corporation). The obtained vector was named pCI-neo(hTfR). Next, the vector was digested with MluI and NotI to cut out a gene fragment encoding hTfR, and the gene fragment was incorporated between MluI and NotI of pE-mIRES-GS-puro, which is an expression vector described in WO2012/063799A, to construct pE-mIRES-GS-puro(hTfR), which is an hTfR expression vector.

### [Example 2] Production of recombinant hTfR

The pE-mIRES-GS-puro(hTfR) was transfected to CHO-K1 cells by an electroporation method, and then the cells were selectively cultured using a CD OPTICHO^{™} culture medium (Invitrogen) containing methionine sulfoximine (MSX) and puromycin to obtain recombinant hTfR-expressing cells. The recombinant hTfR-expressing cells were cultured to prepare a recombinant hTfR (rhTfR).

### [Example 3] Production of phage library

An alpaca was immunized using the rhTfR prepared in Example 2 as an antigen. Blood was collected from the alpaca after immunization, and peripheral blood lymphocytes were isolated from the blood. RNA was extracted from the peripheral blood lymphocytes, and the RNA was reverse-transcribed to obtain cDNA. Using the obtained cDNA as a template, a DNA fragment containing a nucleotide sequence encoding a variable region of a heavy chain antibody was amplified by a PCR method. The amplified DNA fragment was incorporated into a phagemid such that the variable region of the heavy chain antibody was presented on the surface of the phage in a case where the DNA fragment was packaged into the phage, and the phagemid was transfected to Escherichia coli by an electroporation method. The Escherichia coli into which the phagemid had been transfected was further infected with a helper phage (M13K07), and then the Escherichia coli was cultured to release the phage into the culture medium. After completion of the culture, the culture supernatant containing the phage was collected by centrifuging the culture medium. The culture supernatant containing the collected phage was used as a phage library. The concentration of the phage library was 3.36 × 10¹³ PFU/mL.

### [Example 4] Isolation of anti-hTfR heavy chain antibody-expressing phage clones

A phage (anti-hTfR heavy chain antibody expressing phage) expressing a variable region of a heavy chain antibody that specifically binds to hTfR was cloned from the phage library produced in Example 3 by a biopanning method. The cloning was carried out generally as follows. A solution containing rhTfR produced in Example 2 was added to each well of the microtiter plate to immobilize rhTfR on the plate. Next, each well was washed three times with a 0.05% PBST solution, and then a blocking agent was added to each well to block each well. Next, each well was washed three times with a 0.05% PBST solution, and a phage library diluted with a blocking agent was added thereto. The plate was allowed to stand, and a phage having an anti-hTfR heavy chain antibody on the surface was bound to rhTfR. Next, each well was washed 5 times with a 0.05% PBST solution to remove phages that did not bind to rhTfR. Next, the phage bound to rhTfR was dissociated from rhTfR by adding an acidic eluent, and the dissociated phage was collected as a solution.

The collected solution was diluted and added to a solution containing Escherichia coli, and the phage was allowed to infect Escherichia coli. The phage-infected Escherichia coli was further infected with a helper phage (M13K07), and then the Escherichia coli was cultured to release the phage into the culture medium. The phage was purified using a PEG solution.

The purified phage was allowed to infect Escherichia coli, and then the infected Escherichia coli was seeded on a gelatin culture medium. E. coli that formed a single colony on the culture medium was picked up, further cultured in the culture medium, and then infected with a helper phage (M13K07) to release a phage into the culture medium. The phage released into the culture medium was collected in the culture supernatant by centrifuging the culture medium as an anti-hTfR heavy chain antibody-expressing phage clone. Each phage clone contained in the culture supernatant was purified using a PEG solution.

### [Example 5] Determination of amino acid sequence of variable region of anti-hTfR heavy chain antibody expressed by each anti-hTfR heavy chain antibody-expressing phage clone

Two clones were selected from the anti-hTfR heavy chain antibody-expressing phages isolated in Example 4, and each of the clones was named Clone 1 and Clone 2. A phagemid was purified from each clone, and the nucleotide sequence of the variable region of the anti-hTfR heavy chain antibody encoded by each purified phagemid was determined. Based on this, the amino acid sequence of the variable region of the anti-hTfR heavy chain antibody expressed by each clone was determined. Here, the peptide having the variable region of the anti-hTfR heavy chain antibody of Clone 1 was defined as hTFR affinity peptide 1, and the peptide having the variable region of the anti-hTfR heavy chain antibody of Clone 2 was defined as hTFR affinity peptide 3. In addition, hTFR affinity peptide 1 having a humanized amino acid sequence was referred to as hTFR affinity peptide 2. The amino acid sequences of hTFR affinity peptides 1 to 3 are shown in Table 1.

**[Table 1]**

| Table 1 Amino acid sequences of hTFR affinity peptide 1 to 3 | |
|---|---|
| hTFR affinity peptide | Amino acid sequences |
| 1 | SEQ ID NO: 4 |
| 2 | SEQ ID NO: 5 |
| 3 | SEQ ID NO: 6 |

The hTfR affinity peptides 1 to 3 each include CDR1 to 3. The amino acid sequences of the CDRs of the hTfR affinity peptide 1 and the hTfR affinity peptide 2 are the same. Table 2 shows the amino acid sequences of each CDR.

**[Table 2]**

| Table 2 SEQ ID NOs of amino acid sequence included in CDR1 to CDR3 of hTfR affinity peptide 1 to 3 (examples of amino acid sequence of each CDR) | | | |
|---|---|---|---|
| Name | Amino acid sequence | | |
| | CDR1 | CDR2 | CDR3 |
| hTfR affinity peptide 1 | 7, 8 | 9, 10 | 11, 12 |
| hTfR affinity peptide 2 | 7, 8 | 9, 10 | 11, 12 |
| hTfR affinity peptide 3 | 13, 14 | 15, 16 | 17, 18 |

### [Example 6] Production of hTfR affinity peptides 1 and 3

A DNA fragment including a gene encoding the hTfR affinity peptide 1 to which a peptide tag represented by SEQ ID NO: 41 was added at the N-terminus was artificially synthesized, and incorporated between the MluI site and the NotI site of pCI-neo Mammalian Expression Vector (Promega Corporation) to prepare an hTfR affinity peptide 1 expression vector. The expression vector was transfected to CHO-S cells using ExpiCHO Expression System (Thermo Fisher Scientific, Inc.) according to the attached protocol, and hTfR affinity peptide 1 was expressed in the CHO-S cells. This cell expresses the hTfR affinity peptide 1 and secretes the hTfR affinity peptide 1 into the culture medium. This cell was cultured in the presence of neomycin according to the attached protocol. Specifically, CHO-S cells were suspended in 15 mL of a culture medium such that the concentration was 6 × 10⁶ cells/mL, the suspension was transferred to a 150 mL conical flask, and the cells were cultured at 37°C in a wet environment consisting of 5% CO₂ and 95% air at a stirring speed of about 120 rpm for 1 day. The feed liquid and the enhancer liquid attached to the ExpiCHO Expression System were added thereto, and the cells were cultured at 32°C in a wet environment consisting of 5% CO₂ and 95% air at a stirring speed of about 120 rpm for 9 days to express the hTfR affinity peptide 1. After the culture, the culture medium was centrifuged (3,000 xg, 5 minutes) to obtain a supernatant, the supernatant was filtered through a 0.22 µm filter (MilliporeSigma), the filtrate was collected as a culture supernatant. The hTfR affinity peptide 1 was purified from the collected culture supernatant by the following method to obtain a purified product.

As an equilibration solution, 5 column volumes of 50 mM Tris-HCl buffer solution (pH 7.5) containing 150 mM NaCl and 1 mM CaCl₂ was passed through an Anti-FLAG M1 Agarose Affinity Gel column (Sigma-Aldrich Co. LLC). Next, the culture supernatant was applied onto a column, and the hTfR affinity peptide 1 was adsorbed to an affinity column. Next, the column was washed with 5 column volumes of the equilibration solution. The hTfR affinity peptide 1 was eluted from the column with an eluent (50 mM Tris-HCl buffer solution (pH 7.5) containing 150 mM NaCl). The hTfR affinity peptide 1 eluted was used as a purified product in the following experiments. A purified product of the hTfR affinity peptide 3 was also obtained in the same manner as in the hTfR affinity peptide 1.

### [Example 7] Evaluation of binding activity of hTfR affinity peptides 1 and 3 to human TfR, monkey TfR, and mouse TfR

The measurement of the binding activity of the hTfR affinity peptides 1 and 3 to human TfR (hTfR), monkey TfR, and mouse TfR was performed using OctetRED96 (ForteBio, a division of Pall Corporation), which is a biomolecular interaction analysis system using biolayer interferometry (BLI). The basic principle of the biolayer interferometry will be briefly described. In a case where the layer of the biological molecule immobilized on the sensor chip surface is irradiated with light having a specific wavelength, light is reflected from two surfaces of the layer of the biological molecule and the layer serving as a reference inside, and an interference wave of light is generated. By binding the molecules in the measurement sample to the biological molecules on the sensor chip surface, the thickness of the layer at the distal end of the sensor increases, and a wavelength shift in the interference wave occurs. By measuring the change in the wavelength shift, the quantification and kinetic analysis of the number of molecules binding to the biomolecules immobilized on the surface of the sensor chip can be performed in real time. The measurement was generally performed according to the operation manual attached to OctetRED96. As the human TfR, a recombinant hTfR having the amino acid sequence of the extracellular region of hTfR from the cysteine residue at 89th position from the N-terminus to the phenylalanine residue at the C-terminus in the amino acid sequence set forth in SEQ ID NO: 1, to which a histidine tag was added at the N-terminus, was used (rhTfR, Sino Biological Inc.). As the monkey TfR, a recombinant monkey TfR (r monkey TfR, Sino Biological Inc.) having the amino acid sequence of the extracellular region of TfR of cynomolgus monkey from the cysteine residue at 89th position from the N-terminus to the phenylalanine residue at the C-terminus in the amino acid sequence set forth in SEQ ID NO: 2, to which a histidine tag was added at the N-terminus, was used. As the mouse TfR, a recombinant mouse TfR (r mouse TfR) having the amino acid sequence of the extracellular region of mouse TfR from the cysteine residue at 89th position from the N-terminus to the phenylalanine residue at the C-terminus in the amino acid sequence set forth in SEQ ID NO: 3, to which a histidine tag was added at the N-terminus, was used.

The purified products of the hTfR affinity peptides 1 and 3 obtained in Example 6 were diluted with HBS-P+ (10 mM HEPES containing 150 mM NaCl, 1% BSA, 50 µM EDTA, and 0.05% surfactant P20) to prepare solutions having three-stage concentrations of 10 to 40 nM. These solutions were used as sample solutions. The rhTfR, r monkey TfR, and r mouse TfR were each diluted with HBS-P+ to prepare a 15 µg/mL solution, and the solutions were each used as an hTfR-ECD (Histag) solution, a monkey TfR-ECD (Histag) solution, and a mouse TfR-ECD (Histag) solution.

The above-described sample solution was dispensed into 96 well plate, black (Greiner Bio-One) in an amount of 200 µL/well. In addition, the hTfR-ECD (Histag) solution, the monkey TfR-ECD (Histag) solution, and the mouse TfR-ECD (Histag) solution prepared above were dispensed into each predetermined well in an amount of 200 µL/well. HBS-P+ was dispensed into each of the wells for the baseline, the dissociation, and the washing in an amount of 200 µL/well. 10 mM Glycine-HCl (pH 1.7) was dispensed into the wells for regeneration in an amount of 200 µL/well. 0.5 mM NiCl₂ solution containing 1% BSA was dispensed into the wells for activation in an amount of 200 µL/well. The plate and a biosensor (Biosensor/Ni-NTA: ForteBio, a division of Pall Corporation) were installed at predetermined positions of OctetRED96.

OctetRED96 was operated under the conditions shown in Table 3 to acquire data, and then the binding reaction curve was fitted to a 1:1 binding model or a 2:1 binding model using the analysis software attached to OctetRED96 to measure the association rate constant (kon) and the dissociation rate constant (koff) of each hTfR affinity peptide to hTfR, monkey TfR, and mouse TfR, and calculate the dissociation constant (K_{D}). The measurement was performed at a temperature of 25°C to 30°C.

**[Table 3]**

| Table 3 Operating condition of Octet RED96 | | | | |
|---|---|---|---|---|
| | Step | Contact time (sec) | Rate (rpm) | Threshold value |
| 1 | Regeneration | 5 | 1000 | - |
| 2 | Washing | 5 | 1000 | - |

| Steps 1 and 2 are repeated 6 times. | | | | |
|---|---|---|---|---|
| 3 | Activation | 60 | 1000 | 1.0 |
| 4 | Baseline 1 | 120 | 1000 | - |
| 5 | Loading | 600 | 1000 | - |
| 6 | Baseline 2 | 150 | 1000 | - |
| 7 | Association | 120 | 1000 | - |
| 8 | Dissociation | 200 | 1000 | - |
| Steps 1 to 8 are repeated until measurement of all samples is completed. | | | | |

Table 4 shows the dissociation constants (K_{D}) of the hTfR affinity peptides 1 and 3 with hTfR, monkey TfR, and mouse TfR. As a result, the K_{D} values of the hTfR affinity peptide 1 for hTfR, monkey TfR, and mouse TfR were each measured to be 9.32 × 10⁻¹⁰ M, 8.24 × 10⁻⁹ M, and 5.31 × 10⁻⁹ M. In addition, the KD values of the hTfR affinity peptide 3 for hTfR, monkey TfR, and mouse TfR were each measured to be 4.40 × 10⁻⁹ M, 1.74 × 10⁻⁸ M, and 5.75 × 10⁻⁹ M. These results show that each hTfR affinity peptide has a KD value for hTfR of approximately 1.0 × 10⁻⁹ M to 1.0 × 10⁻⁸ M, a KD value for monkey TfR of approximately 5.0 × 10⁻⁹ M to 5.0 × 10⁻⁸ M, and a KD value for mouse TfR of approximately 1.0 × 10⁻⁹ M to 1.0 × 10⁻⁸ M.

**[Table 4]**

| Table 4 Affinity (KD value) of hTfR affinity peptides 1 and 3 for hTfR, monkey TfP, and mouse TfR | | | |
|---|---|---|---|
| Name | Human | Monkey | Mouse |
| hTfR affinity peptide 1 | 9.32 × 10⁻¹⁰ | 8.24 × 10⁻⁸ | 5.31 × 10⁻⁹ |
| hTfR affinity peptide 3 | 4.40 × 10⁻⁹ | 1.74 × 10⁻⁸ | 5.75 × 10⁻⁹ |

From the above results, a relative value of the dissociation constant of each hTfR affinity peptide with the monkey TfR or the mouse TfR in a case where the value of the dissociation constant with the hTfR of each hTfR affinity peptide was set to 1 was calculated (Table 5). The relative affinity of any hTfR affinity peptide to the monkey TfR and the mouse TfR was in a range of 1.0 to 10.0. These results show that the affinity for hTfR and the affinity for the monkey TfR and the mouse TfR are approximately the same. Therefore, since it is naturally predicted that the in vivo pharmacokinetics in a case of being administered to a monkey or a mouse of these hTfR affinity peptides closely approximate the in vivo pharmacokinetics in a case of being administered to a human, in a case of developing a pharmaceutical by applying these, a part of non-clinical trials such as pharmacokinetic tests of the pharmaceutical can be performed using a monkey, a mouse, or both. Therefore, the development of the pharmaceutical product can be significantly promoted.

**[Table 5]**

| Table 5 Relative value of affinity of hTfR affinity peptide 1 and 3 for monkey TfR and mouse TfR | | |
|---|---|---|
| Name | Relative value | |
| | Monkey | Mouse |
| hTfR affinity peptide 1 | 8.84 | 5.70 |
| hTfR affinity peptide 3 | 3.95 | 1.31 |

### [Example 8] Production of hTfR affinity peptide-trastuzumab expression vector

pCI-neo Mammalian Expression Vector (Promega Corporation) was digested with MluI and NotI. A DNA fragment of a gene that has the nucleotide sequence set forth in SEQ ID NO: 34 and encodes a protein having the amino acid sequence set forth in SEQ ID NO: 31 in which the hTfR affinity peptide 1 was bound to the C-terminus of a trastuzumab heavy chain having the amino acid sequence set forth in SEQ ID NO: 29 via a peptide linker having the amino acid sequence set forth in SEQ ID NO: 24, the DNA fragment having a MluI site on the 5' side and a NotI site on the 3' side of the gene, was artificially synthesized. The expression vector of a protein in which the hTfR affinity peptide 1 and the trastuzumab heavy chain were bound to each other was produced by digesting the DNA fragment with MluI and NotI and incorporating the digested DNA fragment between the MluI site and the NotI site of the pCI-neo Mammalian Expression Vector. This expression vector was named pCI-neo-Trastuzumab(HC)-VHH(1). Similarly, for the hTfR affinity peptide 2, a DNA fragment including a gene encoding a protein in which the hTfR affinity peptide 2 was bound to the trastuzumab heavy chain were also artificially synthesized. The DNA fragment was digested with MluI and NotI, and incorporated between the MluI site and the NotI site of the pCI-neo Mammalian Expression Vector, thereby producing an expression vector of a protein in which the hTfR affinity peptide 2 and the trastuzumab heavy chain were bound to each other. The produced expression vector was named pCI-neo-Trastuzumab(HC)-VHH(2). Similarly, for the hTfR affinity peptide 3, a DNA fragment including a gene encoding a protein in which the hTfR affinity peptide 3 was bound to the trastuzumab heavy chain were also artificially synthesized. The DNA fragment was digested with MluI and NotI, and incorporated between the MluI site and the NotI site of the pCI-neo Mammalian Expression Vector, thereby producing an expression vector of a protein in which the hTfR affinity peptide 3 and the trastuzumab heavy chain were bound to each other. The produced expression vector was named pCI-neo-Trastuzumab(HC)-VHH(3).

In addition, a DNA fragment of a gene that has the nucleotide sequence set forth in SEQ ID NO: 35 and encodes the light chain of trastuzumab having the amino acid sequence set forth in SEQ ID NO: 30, the DNA fragment having a MluI site on the 5' side and a NotI site on the 3' side of the gene, was artificially synthesized. The expression vector of the light chain of trastuzumab was constructed by digesting the DNA fragment with MluI and NotI, and ligating the digested DNA fragment with the pCI-neo Mammalian Expression Vector (Promega Corporation) digested with MluI and NotI, which was prepared in advance. The expression vector was named pCI-neo-Trastuzumab(LC).

### [Example 9] Production of hTfR affinity peptide-trastuzumab

pCI-neo-Trastuzumab(HC)-VHH(1) and pCI-neo-Trastuzumab(LC), which were produced in Example 8, were simultaneously transfected to CHO-S cells using ExpiCHO Expression System (Thermo Fisher Scientific, Inc.) according to the attached protocol, and the protein in which the hTfR affinity peptide 1 and the heavy chain of trastuzumab were bound to each other and the light chain of trastuzumab were co-expressed in CHO-S cells. This cell expresses the protein in which the hTfR affinity peptide and trastuzumab are fused to each other, and secretes the protein into a culture medium. Hereinafter, this fusion protein is referred to as hTfR affinity peptide-trastuzumab 1. This cell was cultured in the presence of neomycin according to the attached protocol. Specifically, CHO-S cells were suspended in 15 mL of a culture medium such that the concentration was 6 × 10⁶ cells/mL, the suspension was transferred to a 150 mL conical flask, and the cells were cultured at 37°C in a wet environment consisting of 5% CO₂ and 95% air at a stirring speed of about 120 rpm for 1 day. The feed liquid and the enhancer liquid attached to the ExpiCHO Expression System were added thereto, and the cells were cultured at 32°C in a wet environment consisting of 5% CO₂ and 95% air at a stirring speed of about 120 rpm for 9 days to express the hTfR affinity peptide-trastuzumab 1. After the culture, the culture medium was centrifuged (3,000 xg, 5 minutes) to obtain a supernatant, the supernatant was filtered through a 0.22 µm filter (MilliporeSigma), the filtrate was collected as a culture supernatant.

An open column filled with 1 mL of MabSelect SuRe LX (Cytiva) was equilibrated by passing through 5 column volumes of 25 mM HEPES buffer solution (pH 7.5) containing 50 mM NaCl. Next, the culture supernatant was applied onto a column, and the hTfR affinity peptide-trastuzumab 1 was bound to the resin. The column was washed by passing through 5 column volumes of 25 mM HEPES buffer solution (pH 7.5) containing 50 mM NaCl, and then hTfR affinity peptide-trastuzumab 1 was eluted by passing 150 mM Glycine (pH 3.0) containing 100 mM NaCl. 1 M Tris-HCl (pH 8.0) equivalent to 5% by volume was added to the eluate containing the hTfR affinity peptide-trastuzumab 1 to adjust the pH to neutral. The solvent of the eluate was buffer-exchanged with PBS(-) using a PD-10 column (Cyteva Inc.). The obtained solution was used as a purified product of hTfR affinity peptide-trastuzumab 1 in the following experiments.

In addition, pCI-neo-Trastuzumab(HC)-VHH(2) was also transfected to CHO-S cells simultaneously with pCI-neo-Trastuzumab(LC) as in the case of pCI-neo-Trastuzumab(HC)-VHH(1), and a protein in which the hTfR affinity peptide 2 and the trastuzumab heavy chain were bound and the trastuzumab light chain were co-expressed in CHO-S cells. This cell expresses a protein in which the hTfR affinity peptide 2 and trastuzumab are fused to each other, and secretes the protein into a culture medium. Hereinafter, the fusion protein expressed by this cell is referred to as hTfR affinity peptide-trastuzumab 2. A purified product of the hTfR affinity peptide-trastuzumab 2 was also obtained in the same manner as in the hTfR affinity peptide-trastuzumab 1. In addition, pCI-neo-Trastuzumab(HC)-VHH(3) was also transfected to CHO-S cells simultaneously with pCI-neo-Trastuzumab(LC) as in the case of pCI-neo-Trastuzumab(HC)-VHH(1), and a protein in which the hTfR affinity peptide 3 and the trastuzumab heavy chain were bound and the trastuzumab light chain were co-expressed in CHO-S cells. This cell expresses a protein in which the hTfR affinity peptide 3 and trastuzumab are fused to each other, and secretes the protein into a culture medium. Hereinafter, the fusion protein expressed by this cell is referred to as hTfR affinity peptide-trastuzumab 3. A purified product of the hTfR affinity peptide-trastuzumab 3 was also obtained in the same manner as in the hTfR affinity peptide-trastuzumab 1. The hTfR affinity peptide-trastuzumab 1, 2, and 3 are collectively referred to as hTfR affinity peptide-trastuzumab.

### [Example 10] Evaluation of binding activity of hTfR affinity peptide-trastuzumab to mouse TfR and hTfR

The affinity of the hTfR affinity peptide-trastuzumab 1 and 3 produced in Example 9 for mouse TfR was measured according to the method described in Example 7. As a result, the dissociation constants (K_{D}) for the affinity of the hTfR affinity peptide-trastuzumab 1 and 3 for the mouse TfR were each measured to be 4.03 × 10⁻¹⁰ M and 3.44 × 10⁻¹⁰ M. These results show that the fusion proteins in which different protein were fused to the N-terminus of the hTfR affinity peptides 1 and 3 maintained the affinity for mouse TfR. In addition, the affinity of the hTfR affinity peptide-trastuzumab 2 and 3 produced in Example 9 for hTfR was measured according to the method described in Example 7. As a result, the dissociation constants (K_{D}) for the affinity of the hTfR affinity peptide-trastuzumab 2 and 3 for the hTfR were each measured to be 1.55 × 10⁻¹⁰ M and 2.28 × 10⁻¹⁰ M. These results show that the fusion proteins in which different protein were fused to the N-terminus of the hTfR affinity peptides 2 and 3 maintained the affinity for hTfR.

### [Example 11] Brain transferability evaluation of hTfR affinity peptide-trastuzumab using wild-type mouse

Next, in a case where hTfR affinity peptide-trastuzumab 1 and 3 were each intravenously administered, it was evaluated whether or not the hTfR affinity peptide-trastuzumab 1 and 3 passed through the BBB and were transferred to the brain, using a wild-type mouse (WT mouse).

The purified products of the hTfR affinity peptide-trastuzumab 1 and 3 obtained in Example 9 were each intravenously administered to two male WT mice such that the dose was 5 mg/kg. In addition, as a negative control, trastuzumab was intravenously administered to two male WT mice such that the dose was 5 mg/kg. Furthermore, the hTfR affinity peptide-trastuzumab (reference product) produced in Reference Example 2 was intravenously administered to two male hTfR-KI mice each such that the dose was 5 mg/kg. Six hours after the administration, the mouse was euthanized, and then the peripheral blood was collected, and then the whole body was perfused with physiological saline, and the brain (including the cerebral cortex and the cerebellum) was collected. After measuring the weight (wet weight) of the excised brain, the brain was divided in the sagittal plane, and one part was used for measuring the concentration of the hTfR affinity peptide-trastuzumab in the tissue, and the other part was used for immunohistochemical staining.

The brain tissue for the concentration measurement was homogenized in T-PER (Thermo Fisher Scientific, Inc.) containing Protease Inhibitor Cocktail (Sigma-Aldrich Co. LLC). The obtained homogenate was centrifuged (3,000 ×g, 5 minutes), and the supernatant was collected. The amounts of the hTfR affinity peptide-trastuzumab and trastuzumab contained in the homogenate supernatant were measured by the following method. First, SULFO-Goat Anti Human IgG (h+1) (Bethyl Laboratories, Inc.) (0.5 µg/mL) and Goat Anti Human Kappa Light chain-biotin (IBL) (0.5 µg/mL) were mixed in equal amounts, 25 µL of the mixture was dispensed into a sampling plate (BM6025 test plate 96well V bottom wide well, BM Equipment Co., Ltd.), and the mixture was shaken at room temperature for 1 hour with a microplate mixer to prepare a reaction solution. 150 µL of SuperBlock blocking buffer in PBS (37515, Thermo Fisher Scientific, Inc.) was added to a Streptavidin Gold plate (Meso Scale Diagnostics) and shaken at room temperature for 1 hour using a microplate mixer to block the plate. After washing with PBST, 25 µL of the reaction solution was added thereto, and the mixture was shaken at room temperature for 1 hour using a microplate mixer. Next, 150 µL of Read buffer T (Meso Scale Diagnostics, Ltd.) was added to each well, and the amount of luminescence from each well was measured using a Sector^{™} Imager 6000 reader. A calibration curve was created from the measured value of the standard sample of the hTfR affinity peptide-trastuzumab of a known concentration, and the measured value of each sample was interpolated in the calibration curve to calculate the amount of the hTfR affinity peptide-trastuzumab contained in the brain per gram weight (wet weight) (the concentration of the hTfR affinity peptide-trastuzumab in the brain tissue). The results are shown in Fig. 1. As compared with trastuzumab, the concentrations of the hTfR affinity peptide-trastuzumab 1 and 3 in the brain tissue were each approximately 26 times and approximately 21 times. In addition, even in comparison with the hTfR affinity peptide-trastuzumab (reference product), the concentrations of the hTfR affinity peptide-trastuzumab 1 and 3 in the brain tissue were each approximately 2.0 times and approximately 1.6 times. These results indicate that the hTfR affinity peptide-trastuzumab 1 and 3 remarkably have a property of passing through the BBB and accumulating in the brain tissue.

The immunohistochemical staining of the hTfR affinity peptide in the brain tissue was generally performed by the following procedure. The brain tissue collected for immunohistochemical staining was immersed in an OCT compound (Sakura Finetek Japan Co., Ltd.), rapidly frozen to -80°C using Histo-Tek PINO (Sakura Finetek Japan Co., Ltd.), and a frozen block of the tissue was produced. The frozen block was thinly cut to 4 µm and attached to a MAS-coated slide glass (manufactured by Matsunami Glass Ind., Ltd.). The slide glass was immersed in 4% paraformaldehyde (Wako Chemical Corporation) at 4°C for 5 minutes, and the sample was fixed. Next, the slide glass was immersed in PBS containing 1% BSA to perform blocking. Next, a Goat Anti hIgG-heavy and light chain Antibody (Bethyl Laboratories, Inc.) appropriately diluted was added dropwise to the tissue thin slice and allowed to react for 1 hour. Next, Fluorescein Amplification Working Solution contained in a TSA-Plus Fluorescence Kit (PerkinElmer Inc.) was added dropwise to the tissue thin slice, and the tissue thin slice was shielded from light and allowed to react for 15 minutes. Next, Anti-Fluorescein-HRP contained in the CSA II Biotin-FRee Tyramide Signal Amplification System (Dako) was added dropwise to the tissue thin slice, and allowed to react for 15 minutes. Next, the tissue thin slice was reacted with a DAB substrate (3,3'-diaminobenzidine, Vector Laboratories, Inc.) and color was formed. Next, the tissue thin slice were counterstained with Mayer's hematoxylin (Merck) and dehydrated, cleared, and enclosed, and then observed with an optical microscope.

Fig. 2 is a staining image of the tissue collected 6 hours after the administration of the hTfR affinity peptide-trastuzumab. In the cerebellar tissue of the mouse to which trastuzumab was administered as a negative control, no staining was observed, which indicated that the trastuzumab administered by intravenous injection did not reach the brain parenchyma of the cerebellum and that there was almost no background staining (Fig. 2(a)). On the other hand, in the cerebellar tissue of the mouse to which the hTfR affinity peptide-trastuzumab 1 was administered, staining was observed in the brain parenchyma, and it was shown that the trastuzumab 1 administered by intravenous injection reached the brain parenchyma of the cerebellum (Fig. 2(b)). Staining of the brain parenchyma was also observed in the cerebellar tissue of the mouse to which the hTfR affinity peptide-trastuzumab 3 was administered, and it was shown that the trastuzumab 3, which was administered by intravenous injection, also reached the brain parenchyma of the cerebellum (Fig. 2(c)). In the cerebellar tissue of the mouse to which the hTfR affinity peptide-trastuzumab (reference product) was administered, staining was also observed in the brain parenchyma, and it was shown that the trastuzumab (reference product) administered by intravenous injection also reached the brain parenchyma of the cerebellum (Fig. 2(d)). Here, in a case where the staining intensity of the cerebellar tissue of the mouse to which any one of trastuzumab 1 or 3 was administered is compared with the staining intensity of the cerebellar tissue of the mouse to which trastuzumab (reference product) was administered, it can be easily confirmed that the staining intensity of the cerebellar tissue of the mouse to which any one of trastuzumab 1 or 3 was administered is significantly higher. These results indicate that the hTfR affinity peptide-trastuzumab 1 and 3 significantly reached the brain parenchyma of the cerebellum by passing through the BBB.

### [Example 12] Brain transferability evaluation 1 of hTfR affinity peptide-trastuzumab using hTfR knock-in mouse

Whether or not the hTfR affinity peptide-trastuzumab passes through the BBB and migrates into the brain via binding to hTfR in a case where the hTfR affinity peptide-trastuzumab is administered by intravenous injection, can be evaluated by using an hTfR knock-in mouse (hTfR-KI mouse) in which a gene encoding the extracellular region of a mouse transferrin receptor is substituted with a gene encoding the extracellular region of a human transferrin receptor gene. The hTfR-KI mouse can be produced generally by the following method.

A DNA fragment having a nucleotide sequence set forth in SEQ ID NO: 38, in which a neomycin resistance gene interposed by a loxP sequence is arranged on the 3' side of cDNA encoding a chimeric hTfR in which an intracellular region is an amino acid sequence of mouse hTfR and an extracellular region is an amino acid sequence of human hTfR, is chemically synthesized. This DNA fragment is incorporated into a targeting vector having the nucleotide sequence set forth in SEQ ID NO: 39 as a 5' arm sequence and the nucleotide sequence set forth in SEQ ID NO: 40 as a 3' arm sequence by a normal method, and the targeting vector is transferred into mouse ES cells by an electroporation method. The mouse ES cells after the gene transfer are selectively cultured in the presence of neomycin, and mouse ES cells in which the targeting vector is incorporated into the chromosome by homologous recombination are selected. The obtained gene-recombined mouse ES cells are injected into an 8-cell stage embryo (host embryo) of an ICR mouse, and the embryo is transplanted into a pseudopregnant mouse (recipient mouse) obtained by mating with a mouse in which the vas deferens is ligated. The coat color of the obtained offspring (chimera mouse) is determined, and an individual in which the ES cells contribute to the formation of the living body with high efficiency, that is, an individual having a high ratio of white hair to the whole hair is sorted out. The chimeric mouse individual is crossed with an ICR mouse to obtain an F1 mouse. White F1 mice are sorted, and DNA extracted from the tail tissue is analyzed, and a mouse in which the mouse transferrin receptor gene is replaced with the chimeric hTfR on the chromosome is defined as an hTfR-KI mouse.

The purified product of the hTfR affinity peptide-trastuzumab obtained in Example 9 is intravenously administered to the hTfR-KI mouse according to the method described in Example 11. Thereafter, by performing the evaluation on each tissue collected from the hTfR-KI mouse according to the methods described in Example 11 and Example 12, it is possible to evaluate whether or not the purified product of the hTfR affinity peptide-trastuzumab has reached the brain.

### [Example 13] Brain transferability evaluation of hTfR affinity peptide-trastuzumab using cynomolgus monkey

The brain transferability of the hTfR affinity peptide-trastuzumab can be evaluated using cynomolgus monkeys. The purified product of the hTfR affinity peptide-trastuzumab obtained in Example 9 and trastuzumab are singly intravenously administered to each one male cynomolgus monkey (Macaca fascicularis) such that the dose is 5 mg/kg. In addition, as a control, the same volume of physiological saline is intravenously administered to one male cynomolgus monkey. After 20 minutes, 1 hour, 2 hours, 4 hours, and 8 hours from the administration, peripheral blood is collected, and the concentrations of hTfR affinity peptide-trastuzumab 5 and trastuzumab contained in the peripheral blood are measured by the method described in Example 11. From the measured values, Cmax (µg/mL), AUC0-8hr (µg/hr/mL), and t1/2β (hr) are calculated.

8 hours after the administration, the whole-body of the cynomolgus monkey is perfused with a physiological saline. After the perfusion, the brain tissue including the medulla oblongata and various organs are excised. The concentrations of the hTfR affinity peptide-trastuzumab and trastuzumab contained in the excised brain tissue and various organs are measured by the method described in Example 11.

For the brain tissue, the brain tissue is divided into the cerebral cortex, the cerebellum, the hippocampus, the midbrain, the pons, the medulla oblongata, the caudate nucleus, the putamen, the globus pallidus, the thalamus, the hypothalamus, the cervical spinal cord, the thoracic spinal cord, and the lumbar spinal cord, and the measurement is performed for each of the divided brain tissues. In addition, for the various organs, the retina, the liver, the kidney, the heart (left ventricle), the heart (aorta valve), the lung, the bone marrow, the spleen, the thymus, the testis, the prostate, the quadriceps femoris, the EDL, the soleus muscle, the triceps brachii, and the diaphragm are collected and measured for each.

### [Example 14] Brain transferability evaluation 2 of hTfR affinity peptide-trastuzumab using hTfR knock-in mouse

Whether or not the hTfR affinity peptide-trastuzumab 2 and 3 pass through the BBB and migrate into the brain in a case where the hTfR affinity peptide-trastuzumab 2 and 3 each are administered by intravenous injection, was evaluated by using an hTfR knock-in mouse (hTfR-KI mouse) in which a gene encoding an extracellular region of a mouse transferrin receptor is substituted with a gene encoding an extracellular region of a human transferrin receptor gene.

The purified products of the hTfR affinity peptide-trastuzumab 2 and 3 obtained in Example 9 were intravenously administered to each of 2 to 3 male hTfR-KI mice (20 to 26 weeks of age) such that the dose was 5 mg/kg. In addition, as a negative control, trastuzumab was intravenously administered to two male wild-type mice (WT mice) such that the dose was 5 mg/kg. Six hours after the administration, all the mouse was euthanized, and then the peripheral blood was collected, and then the whole body was perfused with physiological saline, and the brain (including the cerebral cortex and the cerebellum) was collected. After measuring the weight (wet weight) of the excised brain, the brain was divided in the sagittal plane, and one part was used for measuring the concentrations of the hTfR affinity peptide-trastuzumab 2 and 3 in the tissue, and the other part was used for immunohistochemical staining.

The brain tissue for the concentration measurement was homogenized in T-PER (Thermo Fisher Scientific, Inc.) containing Protease Inhibitor Cocktail (Sigma-Aldrich Co. LLC). The obtained homogenate was centrifuged (3,000 ×g, 5 minutes), and the supernatant was collected. The amounts of the hTfR affinity peptide-trastuzumab 2 or 3, or trastuzumab contained in the homogenate supernatant were measured by the following method. First, SULFO-Goat Anti Human IgG (h+1) (Bethyl Laboratories, Inc.) (0.5 µg/mL) and Goat Anti Human Kappa Light chain-biotin (IBL) (0.5 µg/mL) were mixed in equal amounts, 25 µL of the mixture was dispensed into a sampling plate (BM6025 test plate 96well V bottom wide well, BM Equipment Co., Ltd.), and the mixture was shaken at room temperature for 1 hour with a microplate mixer to prepare a reaction solution. 150 µL of SuperBlock blocking buffer in PBS (37515, Thermo Fisher Scientific, Inc.) was added to a Streptavidin Gold plate (Meso Scale Diagnostics) and shaken at room temperature for 1 hour using a microplate mixer to block the plate. After washing with PBST, 25 µL of the reaction solution was added thereto, and the mixture was shaken at room temperature for 1 hour using a microplate mixer. Next, 150 µL of Read buffer T (Meso Scale Diagnostics, Ltd.) was added to each well, and the amount of luminescence from each well was measured using a Sector^{™} Imager 6000 reader. A calibration curve was created from the measured value of the standard sample of the hTfR affinity peptide-trastuzumab 2 or 3, or trastuzumab of a known concentration, and the measured value of each sample was interpolated in the calibration curve to calculate the amount of the hTfR affinity peptide-trastuzumab 2 or 3, or trastuzumab contained in the brain per gram weight (wet weight) (the concentration of the hTfR affinity peptide-trastuzumab 2 or 3, or trastuzumab in the brain tissue). The results are shown in Fig. 3. As compared with trastuzumab, the concentrations of the hTfR affinity peptide-trastuzumab 2 and 3 in the brain tissue were each approximately 16 times and approximately 15 times. These results indicate that the hTfR affinity peptide-trastuzumab has a property of passing through the BBB and accumulating in the brain tissue.

Next, the immunohistochemical staining of the hTfR affinity peptide in the brain tissue was performed by the following procedure. The brain tissue collected for immunohistochemical staining was immersed in an OCT compound (Sakura Finetek Japan Co., Ltd.), rapidly frozen to -80°C using Histo-Tek PINO (Sakura Finetek Japan Co., Ltd.), and a frozen block of the tissue was produced. The frozen block was thinly cut to 4 µm and attached to a MAS-coated slide glass (manufactured by Matsunami Glass Ind., Ltd.). The slide glass was immersed in 4% paraformaldehyde (Wako Chemical Corporation) at 4°C for 5 minutes, and the sample was fixed. Next, the slide glass was immersed in PBS containing 1% BSA to perform blocking. Next, a Goat Anti hIgG-heavy and light chain Antibody (Bethyl Laboratories, Inc.) appropriately diluted was added dropwise to the tissue thin slice and allowed to react for 1 hour. Next, Fluorescein Amplification Working Solution contained in a TSA-Plus Fluorescence Kit (PerkinElmer Inc.) was added dropwise to the tissue thin slice, and the tissue thin slice was shielded from light and allowed to react for 15 minutes. Next, Anti-Fluorescein-HRP contained in the CSA II Biotin-FRee Tyramide Signal Amplification System (Dako) was added dropwise to the tissue thin slice, and allowed to react for 15 minutes. Next, the tissue thin slice was reacted with a DAB substrate (3,3'-diaminobenzidine, Vector Laboratories, Inc.) and color was formed. Next, the tissue thin slice was counterstained with Mayer's hematoxylin (Merck) and dehydrated, cleared, and enclosed, and then observed with an optical microscope.

Fig. 4 shows a staining image of a tissue collected 6 hours after the administration of hTfR affinity peptide-trastuzumab 2 or 3, or trastuzumab. In the cerebellar tissue of the mouse to which trastuzumab was administered as a negative control, no staining was observed, which indicated that the trastuzumab administered by intravenous injection did not reach the brain parenchyma of the cerebellum and that there was almost no background staining (Fig. 4(a)). On the other hand, in the cerebellar tissue of the mouse to which the hTfR affinity peptide-trastuzumab 2 was administered, staining was observed in the brain parenchyma, and it was shown that the trastuzumab 1 administered by intravenous injection reached the brain parenchyma of the cerebellum (Fig. 4(b)). Staining of the brain parenchyma was also observed in the cerebellar tissue of the mouse to which the hTfR affinity peptide-trastuzumab 3 was administered, and it was shown that the trastuzumab 3, which was administered by intravenous injection, also reached the brain parenchyma of the cerebellum (Fig. 4(c)).

### Industrial Applicability

According to one embodiment of the present invention, it is possible to provide a drug capable of exhibiting an effect in the central nervous system by passing through the blood brain barrier, in which a human transferrin receptor affinity peptide is bound to a pharmacologically active substance.

### Reference Signs List

1: blood vessel
2: brain parenchyma

### Sequence Listing Free Text

SEQ ID NO: 1: amino acid sequence of human transferrin receptor
SEQ ID NO: 2: amino acid sequence of cynomolgus monkey transferrin receptor
SEQ ID NO: 3: amino acid sequence of mouse transferrin receptor
SEQ ID NO: 4: amino acid sequence of hTfR affinity peptide 1
SEQ ID NO: 5: amino acid sequence of hTfR affinity peptide 2
SEQ ID NO: 6: amino acid sequence of hTfR affinity peptide 3
SEQ ID NO: 7: amino acid sequence 1 of CDR1 of hTfR affinity peptides 1 and 2
SEQ ID NO: 8: amino acid sequence 2 of CDR1 of hTfR affinity peptides 1 and 2
SEQ ID NO: 9: amino acid sequence 1 of CDR2 of hTfR affinity peptides 1 and 2
SEQ ID NO: 10: amino acid sequence 2 of CDR2 of hTfR affinity peptides 1 and 2
SEQ ID NO: 11: amino acid sequence 1 of CDR3 of hTfR affinity peptides 1 and 2
SEQ ID NO: 12: amino acid sequence 2 of CDR3 of hTfR affinity peptides 1 and 2
SEQ ID NO: 13: amino acid sequence 1 of CDR1 of hTfR affinity peptide 3
SEQ ID NO: 14: amino acid sequence 2 of CDR1 of hTfR affinity peptide 3
SEQ ID NO: 15: amino acid sequence 1 of CDR2 of hTfR affinity peptide 3
SEQ ID NO: 16: amino acid sequence 2 of CDR2 of hTfR affinity peptide 3
SEQ ID NO: 17: amino acid sequence 1 of CDR3 of hTfR affinity peptide 3
SEQ ID NO: 18: amino acid sequence 2 of CDR3 of hTfR affinity peptide 3
SEQ ID NO: 19: amino acid sequence of peptide linker 1
SEQ ID NO: 20: amino acid sequence of peptide linker 2
SEQ ID NO: 21: amino acid sequence of peptide linker 3
SEQ ID NO: 22: amino acid sequence of peptide linker 4
SEQ ID NO: 23: amino acid sequence of peptide linker 5
SEQ ID NO: 24: amino acid sequence of peptide linker 6
SEQ ID NO: 25: amino acid sequence of peptide linker 7
SEQ ID NO: 26: one example of amino acid sequence of Fc region of human IgG
SEQ ID NO: 27: amino acid sequence of human serum albumin
SEQ ID NO: 28: albumin-binding domain of protein derived from Streptococcus strain G418
SEQ ID NO: 29: amino acid sequence of trastuzumab heavy chain
SEQ ID NO: 30: amino acid sequence of trastuzumab light chain
SEQ ID NO: 31: amino acid sequence of fusion protein of hTfR affinity peptide 1 and trastuzumab heavy chain
SEQ ID NO: 32: amino acid sequence of fusion protein of hTfR affinity peptide 2 and trastuzumab heavy chain
SEQ ID NO: 33: amino acid sequence of fusion protein of hTfR affinity peptide 3 and trastuzumab heavy chain
SEQ ID NO: 34: nucleotide sequence of DNA encoding fusion protein of hTfR affinity peptide 1 and trastuzumab heavy chain, synthetic sequence
SEQ ID NO: 35: nucleotide sequence of DNA encoding trastuzumab light chain, synthetic sequence
SEQ ID NO: 36: nucleotide sequence of primer hTfR5', synthetic sequence
SEQ ID NO: 37: nucleotide sequence of primer hTfR3', synthetic sequence
SEQ ID NO: 38: nucleotide sequence of DNA in which neomycin resistance gene interposed by loxP sequences is arranged on 3' side of cDNA encoding chimeric hTfR, synthetic sequence
SEQ ID NO: 39: nucleotide sequence of 5' arm of targeting vector, synthetic sequence
SEQ ID NO: 40: nucleotide sequence of 3' arm of targeting vector, synthetic sequence
SEQ ID NO: 41: peptide tag
SEQ ID NO: 42: amino acid sequence of hTfR affinity peptide 5
SEQ ID NO: 43: amino acid sequence of fusion protein of hTFR affinity peptide 5 and trastuzumab heavy chain
SEQ ID NO: 44: nucleotide sequence of DNA encoding fusion protein of hTFR affinity peptide 5 and trastuzumab heavy chain, synthetic sequence

## Claims

1. A peptide having an affinity for a human transferrin receptor (a human transferrin receptor affinity peptide), , comprising a variable region of a heavy chain antibody having three complementary determining regions of CDR1, CDR2, and CDR3,
wherein the peptide is selected from the group consisting of the following (1) and (2),
(1) a peptide in which the amino acid sequence of CDR1 is the amino acid sequence set forth in SEQ ID NO: 7 or SEQ ID NO: 8, the amino acid sequence of CDR2 is the amino acid sequence set forth in SEQ ID NO: 9 or SEQ ID NO: 10, and the amino acid sequence of CDR3 is the amino acid sequence set forth in SEQ ID NO: 11 or SEQ ID NO: 12, and
(2) a peptide in which the amino acid sequence of CDR1 is the amino acid sequence set forth in SEQ ID NO: 13 or SEQ ID NO: 14, the amino acid sequence of CDR2 is the amino acid sequence set forth in SEQ ID NO: 15 or SEQ ID NO: 16, and the amino acid sequence of CDR3 is the amino acid sequence set forth in SEQ ID NO: 17 or SEQ ID NO: 18.

2. A human transferrin receptor affinity peptide, which is a peptide selected from the group consisting of the following (1) to (3),
(1) a peptide having 80% or more amino acid sequence identity to the amino acid sequence set forth in SEQ ID NO: 4, and having three complementary determining regions of CDR1, CDR2, and CDR3, which have the amino acid sequences shown in (1) of Claim 1,
(2) a peptide having 80% or more amino acid sequence identity to the amino acid sequence set forth in SEQ ID NO: 5, and having three complementary determining regions of CDR1, CDR2, and CDR3, which have the amino acid sequences shown in (1) of Claim 1, and
(3) a peptide having 80% or more amino acid sequence identity to the amino acid sequence set forth in SEQ ID NO: 6, and having three complementary determining regions of CDR1, CDR2, and CDR3, which have the amino acid sequences shown in (2) of Claim 1.

3. The human transferrin receptor affinity peptide according to Claim 2, wherein the amino acid sequence identity is 85% or more.

4. The human transferrin receptor affinity peptide according to Claim 2, wherein the amino acid sequence identity is 90% or more.

5. The human transferrin receptor affinity peptide according to Claim 2, wherein the amino acid sequence identity is 95% or more.

6. The human transferrin receptor affinity peptide according to Claim 1, wherein the peptide includes an amino acid sequence selected from the group consisting of (1) to (3),
(1) the amino acid sequence in which 1 to 10 amino acids in the amino acid sequence set forth in SEQ ID NO: 4 are substituted, deleted, or added, and in which the amino acid sequence of CDR1 is the amino acid sequence set forth in SEQ ID NO: 7 or SEQ ID NO: 8, the amino acid sequence of CDR2 is the amino acid sequence set forth in SEQ ID NO: 9 or SEQ ID NO: 10, and the amino acid sequence of CDR3 is the amino acid sequence set forth in SEQ ID NO: 11 or SEQ ID NO: 12,
(2) an amino acid sequence in which 1 to 10 amino acids in the amino acid sequence set forth in SEQ ID NO: 5 are substituted, deleted, or added, and in which the amino acid sequence of CDR1 is the amino acid sequence set forth in SEQ ID NO: 7 or SEQ ID NO: 8, the amino acid sequence of CDR2 is the amino acid sequence set forth in SEQ ID NO: 9 or SEQ ID NO: 10, and the amino acid sequence of CDR3 is the amino acid sequence set forth in SEQ ID NO: 11 or SEQ ID NO: 12, and
(3) an amino acid sequence in which 1 to 10 amino acids in the amino acid sequence set forth in SEQ ID NO: 6 are substituted, deleted, or added, and in which the amino acid sequence of CDR1 is the amino acid sequence set forth in SEQ ID NO: 13 or SEQ ID NO: 14, the amino acid sequence of CDR2 is the amino acid sequence set forth in SEQ ID NO: 15 or SEQ ID NO: 16, and the amino acid sequence of CDR3 is the amino acid sequence set forth in SEQ ID NO: 17 or SEQ ID NO: 18.

7. The human transferrin receptor affinity peptide according to Claim 6, wherein the number of substituted, deleted, or added amino acids is 1 to 5.

8. The human transferrin receptor affinity peptide according to Claim 6, wherein the number of substituted, deleted, or added amino acids is 1 to 3.

9. The human transferrin receptor affinity peptide according to any one of Claims 1 to 8, wherein the peptide has an affinity for both an extracellular region of a human transferrin receptor and an extracellular region of a monkey transferrin receptor.

10. The human transferrin receptor affinity peptide according to Claim 9, wherein both a dissociation constant with the extracellular region of the human transferrin receptor and a dissociation constant with the extracellular region of the monkey transferrin receptor are 5 × 10⁻¹¹ M to 1 × 10⁻⁸ M.

11. The human transferrin receptor affinity peptide according to Claim 9, wherein both of dissociation constants with the extracellular region of the human transferrin receptor are 5 × 10⁻¹¹ M to 5 × 10⁻⁷ M.

12. The human transferrin receptor affinity peptide according to Claim 9, wherein in a case where a value of a dissociation constant with the extracellular region of the human transferrin receptor is designated as 1, a value of a dissociation constant with the monkey transferrin receptor is 1 to 10.

13. The human transferrin receptor affinity peptide according to Claim 9, wherein in a case where a value of a dissociation constant with the extracellular region of the human transferrin receptor is designated as 1, a value of a dissociation constant with the monkey transferrin receptor is 3 to 10.

14. A human transferrin receptor affinity peptide in which a plurality of the human transferrin receptor affinity peptides according to any one of Claims 1 to 13 are bound, wherein the peptide is selected from the group consisting of the following (1) to (3),
(1) a peptide in which 2 to 10 of the human transferrin receptor affinity peptides according to any one of Claims 1 to 12 are, directly or via a linker, bound,
(2) a peptide in which 2 or 3 of the human transferrin receptor affinity peptides according to any one of Claims 1 to 12 are, directly or via a linker, bound, and
(3) a peptide in which 2 of the human transferrin receptor affinity peptides according to any one of Claims 1 to 12 are, directly or via a linker, bound.

15. The human transferrin receptor affinity peptide according to Claim 14, wherein each of linkers which link a plurality of the human transferrin receptor affinity peptides to each other is a peptide linker consisting of 1 to 60 amino acids.

16. The human transferrin receptor affinity peptide according to Claim 14, wherein the amino acid sequence of the peptide linker is selected from the group consisting of one amino acid, the amino acid sequence Gly-Ser, the amino acid sequence Ser-Ser, the amino acid sequence Gly-Gly-Ser, the amino acid sequence Gly-Gly-Gly, and one of the amino acid sequences set forth in SEQ ID NO: 19, SEQ ID NO: 20, SEQ ID NO: 21, SEQ ID NO: 22, SEQ ID NO: 23, SEQ ID NO: 24, and SEQ ID NO: 25.

17. The human transferrin receptor affinity peptide according to Claim 14, wherein the amino acid sequence of the peptide linker consists of the amino acid sequence in which 2 to 10 amino acid sequences each selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, and SEQ ID NO: 21 are arranged in series.

18. The human transferrin receptor affinity peptide according to Claim 1, wherein the peptide includes the amino acid sequence selected from the group consisting of the amino acid sequences set forth in SEQ ID NO: 4, SEQ ID NO: 5, and SEQ ID NO: 6.

19. A human transferrin receptor affinity peptide which includes the amino acid sequence in which 1 to 10 amino acids in the amino acid sequence selected from the group consisting of the amino acid sequences set forth in SEQ ID NO: 4, SEQ ID NO: 5, and SEQ ID NO: 6 are substituted, deleted, or added.

20. The human transferrin receptor affinity peptide according to Claim 19, wherein the number of substituted, deleted, or added amino acids is 1 to 5.

21. The human transferrin receptor affinity peptide according to Claim 20, wherein the number of substituted, deleted, or added amino acids is 1 to 3.

22. A human transferrin receptor affinity peptide-drug complex in which a drug is bound to the human transferrin receptor affinity peptide according to any one of Claims 1 to 21.

23. The complex according to Claim 22, wherein the drug is any of a different protein (A), a nucleic acid, or a low-molecular-weight compound.

24. A fusion protein of the human transferrin receptor affinity peptide according to any one of Claims 1 to 21 and a different protein (A).

25. The fusion protein according to Claim 24, wherein the human transferrin receptor affinity peptide is bound to the C-terminus of the different protein (A) directly or via a linker.

26. The fusion protein according to Claim 24, wherein the human transferrin receptor affinity peptide is bound to the N-terminus of the different protein (A) directly or via a linker.

27. The fusion protein according to Claim 25 or 26, wherein the linker that links the human transferrin receptor affinity peptide to the different protein (A) is a peptide linker consisting of 1 to 60 amino acids.

28. The fusion protein according to Claim 27, wherein the amino acid sequence of the peptide linker that links the human transferrin receptor affinity peptide to the different protein (A) is selected from the group consisting of one amino acid, the amino acid sequence Gly-Ser, the amino acid sequence Ser-Ser, the amino acid sequence Gly-Gly-Ser, the amino acid sequence Gly-Gly-Gly, and one of the amino acid sequences set forth in SEQ ID NO: 19, SEQ ID NO: 20, SEQ ID NO: 21, SEQ ID NO: 22, SEQ ID NO: 23, SEQ ID NO: 24, and SEQ ID NO: 25.

29. The fusion protein according to Claim 27, wherein the amino acid sequence of the peptide linker that links the human transferrin receptor affinity peptide to the different protein (A) consists of the amino acid sequence in which 2 to 10 amino acid sequences each selected from the group consisting of one amino acid, the amino acid sequence Gly-Ser, the amino acid sequence Ser-Ser, the amino acid sequence Gly-Gly-Ser, the amino acid sequence Gly-Gly-Gly, and the amino acid sequences set forth in SEQ ID NO: 19, SEQ ID NO: 20, and SEQ ID NO: 21 are arranged in series.

30. The fusion protein according to any one of Claims 24 to 29, wherein the different protein (A) is a protein derived from a human.

31. The fusion protein according to any one of Claims 24 to 30, wherein the different protein (A) is a cytokine, a growth factor, or an antibody drug.

32. The fusion protein according to any one of Claims 24 to 30, wherein the different protein (A) is selected from the group consisting of a brain-derived nerve growth factor (BDNF), a nerve growth factor (NGF), a lysosomal enzyme, a ciliary neurotrophic factor (CNTF), a glial cell line neurotrophic factor (GDNF), a neurotrophin 3, a neurotrophin 4/5, a neurotrophin 6, a neuregulin 1, an erythropoietin, a darbepoietin, an activin, a basic fibroblast growth factor (bFGF), a fibroblast growth factor 2 (FGF2), an epidermal growth factor (EGF), a vascular endothelial growth factor (VEGF), an interferon α, an interferon β, an interferon γ, an interleukin 6, a granulocyte-macrophage colony-stimulating factor (GM-CSF), a granulocyte colony-stimulating factor (G-CSF), a macrophage colony-stimulating factor (M-CSF), a tumor necrosis factor α receptor (TNF-α receptor), a PD-1 ligand, a PD-L1, a PD-L2, an enzyme having an activity of degrading β-amyloid, an anti-β-amyloid antibody, an anti-BACE antibody, an anti-EGFR antibody, an anti-PD-1 antibody, an anti-PD-L1 antibody, an anti-PD-L2 antibody, an anti-HER2 antibody, an anti-TNF-α antibody, and an anti-CTLA-4 antibody.

33. The fusion protein according to any one of Claims 24 to 30, wherein the different protein (A) is a lysosomal enzyme, and
wherein the lysosomal enzyme is selected from the group consisting of α-L-iduronidase, iduronate-2-sulfatase, acidic α-glucosidase, glucocerebrosidase, β-galactosidase, GM2-activator protein, β-hexosaminidase A, β-hexosaminidase B, N-acetylglucosamine-1-phosphotransferase, α-mannosidase, β-mannosidase, galactosylceramidase, saposin C, arylsulfatase A, α-L-fucosidase, aspartylglucosaminidase, α-N-acetylgalactosaminidase, acidic sphingomyelinase, α-galactosidase A, β-glucuronidase, heparan N-sulfatase, α-N-acetylglucosaminidase, acetyl CoA α-glucosaminide N-acetyltransferase, N-acetylglucosamine-6-sulfatase, acidic ceramidase, amylo-1,6-glucosidase, sialidase, palmitoyl-protein thioesterase-1, tripeptidyl peptidase-1, hyaluronidase-1, CLN1, and CLN2.

34. The fusion protein according to any one of Claims 24 to 33, wherein serum albumin is bound to the fusion protein.

35. The fusion protein according to any one of Claims 24 to 33, wherein a human IgG Fc region or a part thereof is bound to the fusion protein.

36. A nucleic acid encoding the human transferrin receptor affinity peptide according to any one of Claims 1 to 21.

37. A nucleic acid encoding the fusion protein according to any one of Claims 24 to 35.

38. An expression vector comprising the nucleic acid according to Claim 36 or 37.

39. A cell transformed with the expression vector according to Claim 38.

40. The cell according to Claim 39, wherein the cell is derived from a mammal.
